(19) **Europäisches Patentamt / European Patent Office / Office européen des brevets**

(11) **EP 3 213 051 B1**

(12) **FASCICULE DE BREVET EUROPEEN**

(45) Date de publication et mention de la délivrance du brevet:
**17.02.2021 Bulletin 2021/07**

(51) Int Cl.:
*G01N 21/3504* (2014.01)    *G01N 33/28* (2006.01)
*G01N 21/3577* (2014.01)    *G01N 21/359* (2014.01)

(21) Numéro de dépôt: **15787552.7**

(22) Date de dépôt: **27.10.2015**

(86) Numéro de dépôt international:
**PCT/EP2015/074876**

(87) Numéro de publication internationale:
**WO 2016/066646 (06.05.2016 Gazette 2016/18)**

(54) **PROCEDE DE DETERMINATION DE L'ORIGINE D'UN MELANGE DE CONSTITUANTS PAR ANALYSE SPECTRALE**

VERFAHREN ZUM BESTIMMEN DES URSPRUNGS EINER MISCHUNG VON BESTANDTEILEN DURCH SPEKTRALANALYSE

METHOD FOR DETERMINING THE ORIGIN OF A MIXTURE OF COMPONENTS BY SPECTRAL ANALYSIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.10.2014 EP 14290326**

(43) Date de publication de la demande:
**06.09.2017 Bulletin 2017/36**

(73) Titulaire: **Topnir Systems SAS
13100 Aix en Provence (FR)**

(72) Inventeurs:
- **LAMBERT, Didier
  F-33430 Bernos Beaulac (FR)**
- **SAINT MARTIN, Claude
  F-13330 Pelissane (FR)**
- **SANCHEZ, Miguel
  F-13117 Lavera (FR)**
- **RIBERO, Bernard
  F-13660 Peyrolles en Provence (FR)**

(74) Mandataire: **De Kezel, Eric et al
Mathisen & Macara LLP
Communications House
South Street
Staines-upon-Thames
Middlesex, TW18 4PR (GB)**

(56) Documents cités:
**WO-A1-2006/126978    FR-A1- 2 619 624
US-A- 5 717 209    US-A- 6 070 128
US-A- 6 087 662    US-A1- 2004 033 617
US-A1- 2010 116 991    US-A1- 2010 307 740**

## Description

La présente invention concerne un procédé de détermination de l'origine d'un mélange de constituants par analyse spectrale. En particulier, la présente invention concerne un procédé pour déterminer la concentration et l'origine de gaz et/ou de pétroles bruts dans une zone de mélange après mélange par transport des dits gaz et/ou pétroles bruts provenant d'au moins deux origines d'extraction différentes, le dit procédé comprenant une analyse spectrale particulière.

Afin de réduire leurs coûts, les pétroliers ont l'habitude de partager leurs installations et équipements, en particulier dans le domaine du transport de gaz et/ou de pétrole. Ainsi, il est bien connu de transporter des mélanges de gaz et/ou de pétrole provenant de différentes sources d'extraction, par exemple de différents puits et/ou de différents champs pétroliers.

Toutefois, il est également crucial pour les pétroliers de pouvoir déterminer de manière précise et efficace la provenance exacte de ces gaz et/ou pétroles après mélange de ces derniers.

US2010116991 concerne une méthode de mesure de la concentration en biodiesel dans un mélange biodiesel-diesel homogène en utilisant une mesure d'un pic d'absorption dans le domaine infrarouge qui correspond au pic d'absorption du groupe carbonyle (CO) qui est seulement présent dans le biodiesel. Cette méthode nécessite une étape de calibration consistant à effectuer dans toute une gamme de concentrations des mélanges diesel/biodiesel et à les analyser de manière à pouvoir ensuite associer à un pic d'absorption du groupe carbonyle (CO) la concentration en biodiesel dans le diesel.

US6087662 décrit une méthode de mesure de la concentration en asphaltènes d'une charge d'hydrocarbures par spectroscopie infrarouge. Cette méthode implique une méthode d'analyse chimique de la teneur en asphaltènes de nombreux échantillons connus pour pouvoir établir des bases de données permettant ensuite de déterminer la concentration en asphaltènes de nouveaux échantillons.

WO2011073855 décrit cette importance et revendique un procédé d'analyse en temps réel de l'affectation de l'emplacement du puits de production ; le procédé consiste à faire des mesures spectroscopiques in situ au voisinage de l'emplacement du puits d'un fluide produit à partir d'un ou plusieurs trous de forage, le dit fluide provenant d'un mélange, d'au moins un premier composant d'une première zone de production et d'un second composant d'une deuxième zone de production, et d'estimer ainsi la répartition réelle d'au moins le premier composant en fonction des mesures spectroscopiques in-situ. La mesure in situ peut être de plusieurs types, par exemple: (1) l'absorption des rayonnements de longueurs d'ondes électromagnétiques dans le domaine de l'ultraviolet, visible et/ou infrarouge, (2) les mesures de spectroscopie de fluorescence par rayons X, (3) des mesures spectroscopiques par diffusion électromagnétique telles que la spectroscopie Raman, (4) des mesures de spectroscopie par résonance magnétique, et (5) des mesures de spectroscopie dans le domaine térahertz.

US2010116991 concerne une méthode de mesure de la concentration en biodiesel dans un mélange biodiesel-diesel homogène en utilisant une mesure d'un pic d'absorption dans le domaine infrarouge qui correspond au pic d'absorption du groupe carbonyle (CO) qui est seulement présent dans le biodiesel. Cette méthode nécessite une étape de calibration consistant à effectuer dans toute une gamme de concentrations des mélanges dicsel/biodicscl et à les analyser de manière à pouvoir ensuite associer à un pic d'absorption du groupe carbonyle (CO) la concentration en biodiesel dans le diesel.

US6087662 décrit une méthode de mesure de la concentration en asphaltènes d'une charge d'hydrocarbures par spectroscopie infrarouge. Cette méthode implique une méthode d'analyse chimique de la teneur en asphaltènes de nombreux échantillons connus pour pouvoir établir des bases de données permettant ensuite de déterminer la concentration en asphaltènes de nouveaux échantillons.

US 6,070,128 D1 concerne un procédé de détermination des propriétés de produits hydrocarbonés par spectroscopie proche infrarouge.

US 2004/0033617 A1 concerne l'analyse des produits pétroliers en effectuant une analyse topologique dans le domaine proche infrarouge.

La présente invention comprend dans certains modes de réalisation un objectif similaire de détermination de manière précise et efficace de la provenance exacte de gaz et/ou pétroles après mélange de ces derniers.

Ainsi, la présente invention concerne un procédé pour déterminer la concentration et l'origine de gaz et/ou de pétroles dans une zone de mélange après mélange par transport des dits gaz et/ou pétroles provenant d'au moins deux origines différentes, selon la revendication 1.

L'origine des gaz et/ou pétroles dans le cadre de la présente invention peut signifier toute sorte de provenance précédant l'étape de mélange des dits gaz et/ou pétroles. En particulier, et ceci constitue un mode d'exécution préféré selon la présente invention, la dite origine constitue une origine d'extraction du dit gaz et/ou pétrole.

Les analyses spectrales selon la présente invention sont effectuées dans le domaine proche infrarouge (« NIR »).

Les analyses spectrales selon la présente invention sont de type analyses spectrales topologiques comme expliquées dans le détail dans la présente description.

L'analyse spectrale de chaque gaz et/ou pétrole provenant de différentes origines peut être effectuée à n'importe quelle localisation an amont de la zone de mélange. On citera à titre d'exemple illustratif la conduite d'acheminement du dit gaz et/ou pétrole, la plate-forme d'extraction, la tête de puits, ou encore à l'intérieur du puits d'extraction ; pour l'analyse spectrale effectuée in situ (comme par exemple la tête de puits ou l'intérieur du puits d'extraction), on pourra avantageusement utiliser une fibre optique. Cette analyse spectrale peut se faire de manière continue (par exemple à la fréquence de la mesure des spectres) et/ou de préférence de manière discontinue (par exemple au moins une fois par jour).

L'analyse spectrale du mélange, celle qui est donc effectuée dans le même domaine de longueurs d'ondes que celles des gaz et/ou pétroles d'origines différentes, peut être effectuée à n'importe quelle localisation an aval du mélange proprement dit. On citera à titre d'exemple illustratif toute conduite d'acheminement du dit mélange de gaz et/ou pétroles. Cette analyse spectrale peut se faire de manière discontinue (par exemple au moins une fois par jour) et/ou de préférence de manière continue (par exemple à la fréquence de la mesure des spectres).

A titre illustratif, pour la mise en évidence du domaine spectral caractérisant et discriminant, on peut avantageusement effectuer comme décrit plus avant dans la description, une analyse statistique des spectres relevés pour chaque échantillon d'une pluralité d' échantillons des gaz et/ou pétroles provenant des différentes origines et pour le gaz et/ou pétrole du mélange.

Selon un mode d'exécution de la présente invention, le domaine spectral caractérisant comprend au moins une donnée spectrale caractérisante (grandeur spectrale caractérisante) et/ou au moins un spectre d'analyse caractérisant et/ou au moins une banque de données spectrales caractérisante, qui discrimine entre elles les différentes origines des gaz et/ou pétroles.

Le domaine spectral caractérisant peut être déterminé par toute méthode d'analyse spectrale appropriée.

On citera à titre d'exemple d'analyse spectrale les analyses RMN, Raman, IR, et/ou UV/Visible, de préférence l'analyse spectrale (topologique) dans le proche infrarouge (« NIR »).

Selon un mode d'exécution préféré de la présente invention, les données spectrales sont des données mesurées au moyen du même type d'analyse spectrale, de préférence au moyen du même type de spectromètre ; ces données spectrales peuvent par exemple être des « spectres ».

Le domaine spectral caractérisant peut être déterminé par toute méthode appropriée. A titre d'exemple, ce domaine sera déterminé aux moyen des agrégats tels que décrits ci-après dans la description. La caractéristique fondamentale du domaine spectral caractérisant est qu'il discrimine entre elles les différentes origines des gaz et/ou pétroles.

Comme déjà indiqué, les données spectrales sont de préférence des données mesurées au moyen du même type d'analyse spectrale, de préférence au moyen du même type de spectromètre ; ces données spectrales peuvent par exemple être tout type approprié de grandeurs spectrales constitutives d'une banque de données spectrales correspondante. Ces grandeurs spectrales peuvent être tous types de signaux caractérisant les spectres, par exemple les absorbances, transmittances, réflectances, etc... ; les absorbances ou densités optiques étant les signaux les plus communément utilisés. A titre d'exemple, on citera également comme signaux les dérivées des absorbances ou encore tout autre mesure résultant d'un autre type de traitement mathématique des dites absorbances.

L'analyse spectrale topologique dans le domaine du proche infrarouge (« NIR ») s'est avérée particulièrement efficace pour permettre la caractérisation et la discrimination des origines (d'extraction) des gaz et/ou pétroles dans le mélange transporté. Dans un exemple, qui ne fait pas parti de l'invention, la détermination du domaine spectral caractérisant pourra également s'effectuer au moyen d'une méthode d'analyse par régression des moindres carrés partiels (PLS).

Le brevet EP0742900 de la Demanderesse constitue la référence du domaine en matière d'analyse spectrale topologique. Il y est décrit une méthode de détermination ou de prédiction d'une valeur Px, d'une propriété d'une matière X ou d'une propriété d'un produit résultant d'un procédé provenant de ladite matière ou du rendement dudit procédé, méthode qui consiste à mesurer l'absorption $D_ix$ de ladite matière à plus d'une longueur d'onde dans la région des 600 à 2 600 nm, à comparer les signaux indicateurs de ces absorptions ou leurs fonctions mathématiques avec des signaux indicateurs des absorptions Dim aux mêmes longueurs d'onde ou leurs fonctions mathématiques pour un certain nombre d'étalons S dans une banque pour lesquels on connaît ladite propriété ou rendement P, et à choisir dans la banque au moins un et de préférence au moins 2 étalons Sm ayant la propriété Pm, ledit étalon Sm ayant les valeurs moyennes les plus petites des valeurs absolues de la différence à chaque longueur d'onde i comprise entre le signal pour la matière et le signal pour l'étalon Sm en vue d'obtenir la valeur Px et à faire la moyenne desdites propriétés ou rendements Pm, lorsqu'on choisit plus d'un étalon Sm.

L'analyse spectrale topologique présente de nombreux avantages par rapport aux méthodes mathématiques régressives classiques. Les méthodes numériques décrites pour la modélisation des propriétés physico-chimiques de substances à base d'analyse spectrale sont de nature corrélative et impliquent des relations à caractère de régression entre la (ou les) propriété(s) étudiée(s). Parmi ces analyses à multiples variables on trouve la régression multilinéaire (MLR), la régression sur composant principal (PLR), la régression canonique et la régression des moindres carrés partiels(PLS). Dans tous les cas, on cherche une relation entre la propriété et le spectre qui peut être linéaire mais qui est habituellement quadratique ou d'une forme algébrique supérieure comportant des coefficients de régression appliqués à chaque absorption. Hors, l'établissement de toute régression demande un étalonnage progressif, puisque l'approche est empirique et n'est pas soutenue par une théorie.

Ces techniques ont des inconvénients, dont le principal est la nécessité d'établir une corrélation forte entre le spectre et la propriété, et leur difficulté à traiter la synergie positive ou négative entre les composants contribuant à cette propriété. Par exemple, pour déterminer la composition chimique par exemple LINA (linéaire, isoparaffinique, naphténique, aromatique) dans une charge d'hydrocarbure alimentant un reformeur catalytique, on a décrit l'utilisation d'une technique PLS sur la base de spectres NIR. Le modèle marche bien sur l'ensemble d'étalonnage mais la réponse des modèles lorsqu'on ajoute des hydrocarbures purs, par exemple du cyclohexane, n'est pas satisfaisante, puisque le modèle prédit des variations en teneur d'isoparaffines et de naphtènes inverses de celles trouvées expérimentalement. De plus, il existe d'autres difficultés pratiques, principalement dues à la nécessité d'identifier des échantillons de familles ayant le même type de relation entre les spectres et les propriétés à modéliser. Ainsi, le modèle peut être limité, en particulier avec une relation non linéaire entre le spectre et la propriété. Surtout lorsqu'aux limites des données disponibles, la précision du modèle se réduit. La stabilité du modèle est également un problème, ainsi que la nécessité lors de l'addition de nouveaux étalons, de réaliser des révisions laborieuses pour obtenir le nouveau modèle, en particulier lorsqu'on ajuste a une nouvelle charge alimentant un procédé; ainsi le contrôle de 6 propriétés sur 4 produits sortant d'une unité de distillation demande 24 modèles, dont chacun doit être modifié pour chaque modification de la charge d'alimentation non comprise dans l'étalonnage. Un autre inconvénient majeur rencontré par ces techniques apparait lorsqu'un point à analyser se situe en dehors du modèle préalablement établi ; il faut alors générer une nouvelle base de données et un nouveau modèle par propriété ce qui rend ce type de technique non seulement peu réactive mais également nécessitant un nombre d'heures de travail beaucoup trop important.

Il faut noter que l'analyse spectrale topologique en tant que telle n'a pas réellement évolué depuis le brevet EP0742900 de la Demanderesse. Toutefois, la présente invention apporte également de nombreuses améliorations à la dite méthode d'analyse spectrale topologique. Les caractéristiques de cette méthode d'analyse spectrale topologique ainsi que ses améliorations et avantages seront décrits dans leurs détails dans la description qui va suivre ainsi que dans les exemples, figures et revendications. D'autres buts et avantages de la présente invention apparaîtront au cours de la description qui va suivre se rapportant à des modes de réalisation qui ne sont donnés qu'à titre d'exemples indicatifs et non limitatifs.

La compréhension de cette description sera facilitée en se référant aux figures 1 à 10 jointes en annexe et dans lesquelles :

- la figure 1 représente le spectre NIR d'un étalon,
- la figure 2 représente un exemple de banque de données spectrales A,
- la figure 3 représente un exemple de banque de données spectrales B (mise en évidence des longueurs d'onde polluantes),
- la figure 4 représente un exemple de banque de données spectrales améliorée A' (banque de données spectrales A dans laquelle on a supprimé les données spectrales correspondant aux longueurs d'onde polluantes),
- la figure 5 représente un exemple de banque de données spectrales élargie E (banque de données spectrales A ou A' dans laquelle on a ajouté des intergermes),
- la figure 6 représente un exemple de banque de données spectrales élargie EE (banque de données spectrales A et/ou E dans laquelle on a ajouté des extragermes),
- la figure 7 représente un exemple de banque de données spectrales élargie EEI (banque de données spectrales E et/ou EE dans laquelle on a ajouté des extragermes'),
- les figures 8 et 9 représentent respectivement un graphique et un tableau représentants des agrégats discriminants, et
- la figure 10 représente une banque de données spectrales du type de celle de la figure 5 dans laquelle ont été ajoutées les caractérisations mesurées des étalons et calculs des intergermes.

En particulier, toutes les approches chémométriques d'analyses spectrales de l'art antérieur requièrent l'établissement d'une banque de données spectrales constituée à partir d'un nombre initial très important d'échantillons et/ou étalons. Bien que l'art antérieur cite des constitutions de banque de données spectrales basées à partir d'au moins 60 ou au moins 100 échantillons et/ou étalons, tous les exemples décrivent des banques constituées d'un nombre d'échantillons nettement supérieurs. Ce nombre est encore plus grand dans les approches chémométriques utilisant les méthodes mathématiques régressives dont les banques de données sont constituées à partir de centaines voire milliers d'échantillons et/ou étalons. La présente invention, dans un mode particulier d'exécution, permet de s'affranchir de cette exigence antérieure ce qui ouvre un nombre considérable de nouvelles applications comme démontré ci-après.

Ainsi, dans un mode particulier d'exécution, dans un premier temps, le procédé selon la présente invention consiste en la préparation d'une banque de spectres et/ou de données spectrales des gaz et/ou pétroles et de leurs constituants, de préférence d'une banque de spectres et/ou de données spectrales élargie E pour un nombre limité de matières d'étalons disponibles (et représentant donc les gaz et/ou pétroles et/ou leurs constituants en fonction de leurs origines d'extraction).

La présente invention s'applique donc plus particulièrement à la spectroscopie du proche infrarouge (NIR). En effet, la spectroscopie NIR présente de nombreux avantages par rapport à d'autres procédés d'analyse, par exemple, dans les raffineries, les sites pétrochimiques ou chimiques ainsi que tous les domaines où la caractérisation de produits chimiques, par exemple les hydrocarbures, en particulier les carburants, et elle peut recouvrir un grand nombre d'applications répétitives avec précision, avec rapidité et en ligne. De plus, la région des NIR entre 800 et 2500 nm contient la totalité des informations moléculaires sous forme de combinaisons et d'harmoniques de vibrations polyatomiques.

Dans une première étape, on effectue un type sélectionné d'analyse spectrale sur chacun des étalons (représentatifs de chacun des gaz et/ou pétroles et/ou de leurs constituants) et on procède au peuplement de la banque de spectres et/ou de données spectrales A en y enregistrant les spectres NIR (par exemple sous forme numérisée ou digitalisée), à plusieurs longueurs d'onde (ou nombres d'onde), par exemple pour un nombre limité de matières d'étalons disponibles.

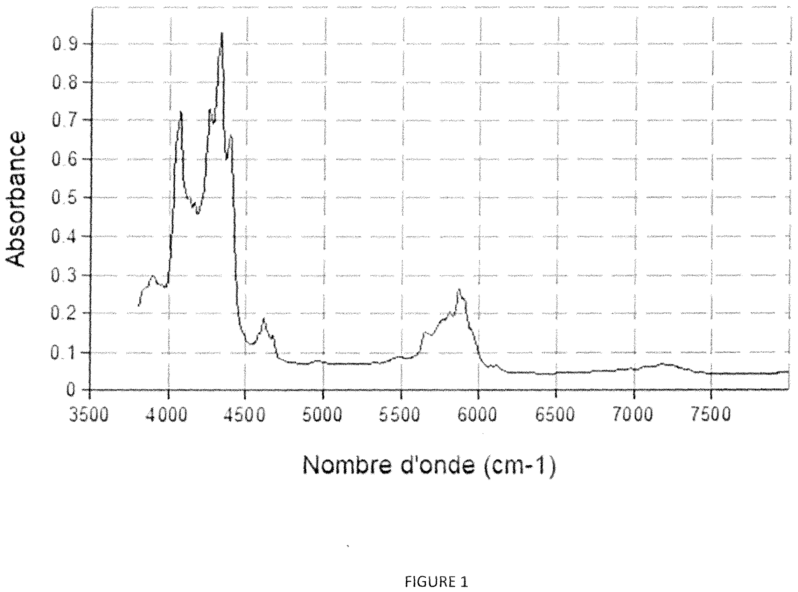

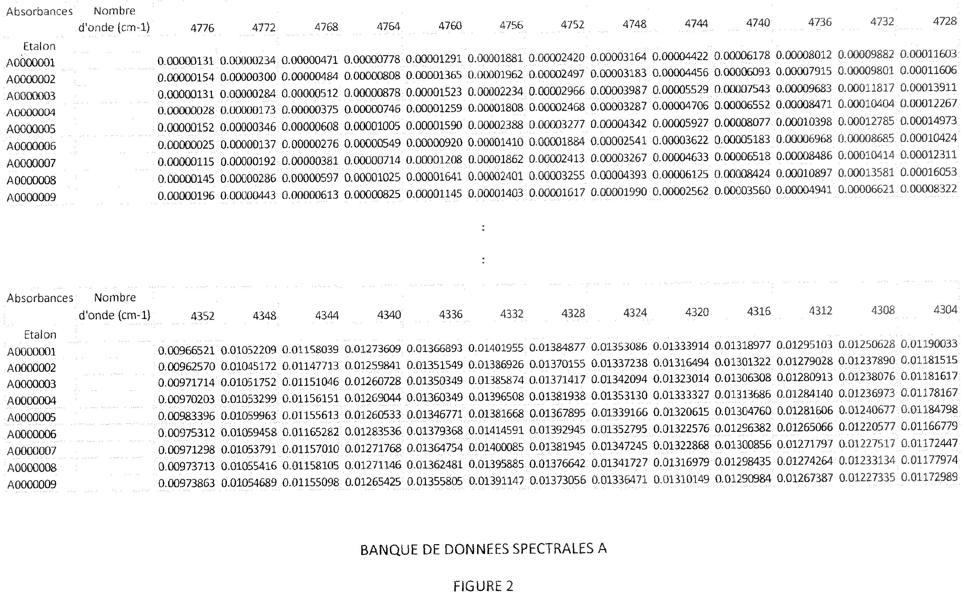

Un exemple de constitution et de représentation de cette banque de données spectrales initiale est décrit au moyen des figures 1 et 2.

La figure 1 représente le spectre NIR d'un étalon sur lequel on peut visualiser comme grandeur spectrale l'absorbance mesurée en fonction du nombre d'onde. Des spectres similaires sont donc également établis de manière identique pour chaque étalon. Dans le présent exemple de représentation, neuf étalons ont été analysés. A partir de ces spectres, on établit un tableau (la banque de données spectrales A) dont un exemple de représentation est donné dans la figure 2 pour un nombre limité de nombres d'onde.

Ainsi, dans le tableau de la figure 2 (qui correspond donc en une vue tronquée - on a représenté deux parties du tableau à des nombres d'onde sélectionnés différents), on peut apercevoir dans la colonne de gauche les références permettant d'identifier les neuf étalons et dans la première ligne la valeur des nombres d'onde ou gammes de nombres d'onde ; le contenu du tableau indique donc les valeurs des grandeurs spectrales (dans le cas présent, les absorbances) qui correspondent au couple « référence étalon / nombre d'onde ». Ces grandeurs spectrales peuvent être tous types de signaux caractérisant les spectres, par exemple les absorbances, transmittances, réflectances, etc... ; les absorbances ou densités optiques étant les signaux les plus communément utilisés. A titre d'exemple, on citera également comme signaux les dérivées des absorbances ou encore tout autre mesure résultant d'un autre type de traitement mathématique des dites absorbances.

Le nombre limité d'étalons disponibles est généralement dicté par le client et/ou l'utilisateur final, désireux d'utiliser des méthodes de contrôle réactives et fiables tout en limitant la nécessité de devoir disposer au préalable d'une grande quantité d'étalons et de devoir en effectuer une analyse selon des méthodes conventionnelles.

Une caractéristique de ce procédé optionnel selon la présente invention est qu'il permet donc de s'affranchir du besoin dicté par l'art antérieur de disposer d'un nombre très important d'étalons. Par exemple, la présente invention permet de caractériser un gaz et/ou pétrole (et son origine) échantillon à partir d'un nombre d'étalons disponibles inférieur à 100, voire inférieur à 60 ou même inférieur à 50. Des résultats très probants ont même pu être obtenus au moyen de la présente invention à partir de moins de 40 étalons disponibles, voire moins de 30 ou même moins de 20. Un minimum de 10 étalons disponibles est toutefois préféré même si la présente invention a déjà été utilisée avec succès avec un minimum de 5 étalons disponibles.

Pour la présente invention, la description qui en est faite et les revendications ci-après, il est évident pour l'homme du métier que les spectres peuvent être réalisés en fonction des longueurs d'onde (et/ou gammes de longueurs d'onde) et/ou en fonction des nombres d'onde (et/ou gammes de nombres d'onde), car le nombre d'onde est représenté par l'inverse de la longueur d'onde.

Pour la présente invention, la description qui en est faite et les revendications ci-après, les étalons seront aussi bien qualifiés de « germes » [« G »], les deux termes étant interchangeables.

Une seconde étape optionnelle et préférée selon la présente invention consiste ensuite en l'élimination des longueurs d'onde et/ou gammes de longueurs d'onde « polluantes » de la banque de données spectrales A. Cette étape consiste

1. à répéter au moins deux fois, de préférence au moins trois fois, plus préférentiellement au moins cinq fois la même analyse spectrale que celle effectuée lors de la première étape, et ceci sur au moins un des étalons disponibles, de préférence sur au moins deux ou même sur la totalité des dits étalons ;
2. à construire une banque de données spectrales B à partir des mesures effectuées au point 1 ci-dessus ;
3. à calculer pour chaque étalon sélectionné au point 1 ci-dessus et pour chaque longueur d'onde et/ou gamme de longueur d'onde (de la banque de données spectrales A) les écarts-type ($\sigma$) des mesures enregistrées dans la banque de données B ;
4. à identifier dans la banque de données B les longueurs d'onde et/ou gamme de longueur d'onde pour lesquelles l'écart-type est supérieur à une valeur prédéterminée ;

5. à supprimer de la banque de données spectrales A les mesures correspondant aux longueurs d'onde identifiées au point 4 ci-dessus.

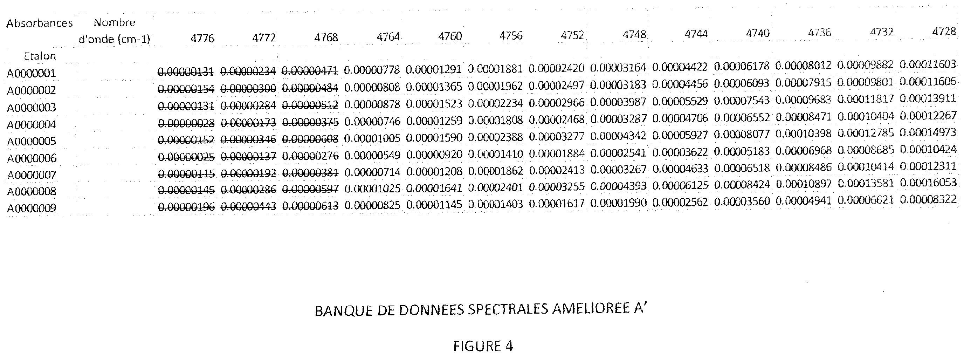

Ainsi, selon un mode d'exécution préféré de la présente invention, l'utilisation de la seconde étape ci-dessus permet l'obtention d'une banque de données spectrales améliorée A' ; un exemple de banque de données spectrales améliorée A' est représentée dans la figure 4.

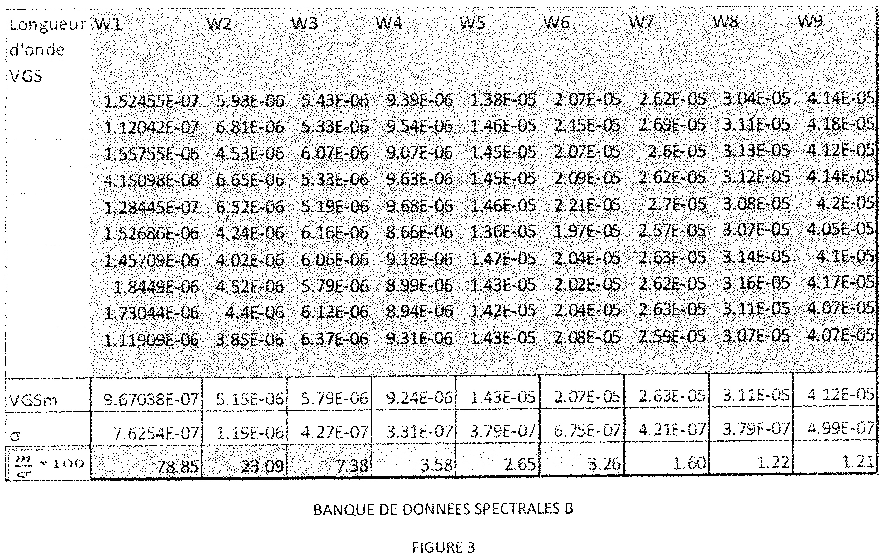

Un exemple de représentation de la banque de données spectrales B est illustré dans la figure 3 par un tableau.

On peut y voir que la même analyse spectrale a été répétée dix (10) fois sur le même échantillon et que les valeurs de grandeurs spectrales correspondantes ont été enregistrées dans le tableau. Les trois dernières lignes du tableau correspondent respectivement et successivement à

- la valeur de grandeur spectrale moyenne VGSmoyenne (« VGSm ») qui correspond à la somme des valeurs de grandeur spectrale divisée par le nombre (« n ») d'analyses effectuées (VGSm = [$\Sigma$ VGS]/n), avec n=10 dans la présente représentation ;
- l'écart-type (« $\sigma$ ») qui correspond à la différence entre VGSmax et VGSmin dans chaque colonne du tableau ;
- le ratio ($\sigma$/(VGSm/100)) dont la valeur (en pourcentage) est calculée en divisant l'écart-type par la valeur de la grandeur spectrale moyenne, le résultat étant multiplié par cent.

Ainsi, la dernière ligne du tableau permet d'identifier dans la banque de données B les longueurs d'onde et/ou gammes de longueurs d'onde pour lesquelles le ratio ($\sigma$/(VGSm/100)) est supérieur à une valeur prédéterminée. Selon un mode d'exécution de la présente invention, on identifie dans le tableau B les colonnes (les longueurs d'onde et/ou gammes de longueurs d'onde) pour lesquelles la valeur des ratios ($\sigma$/(VGSm/100)) est supérieure à 2% (de préférence supérieure à 1,5% ou même 1%) ; ensuite, on élimine de la banque de données A les dites colonnes, à savoir les valeurs de grandeurs spectrales correspondantes aux longueurs d'onde « polluantes ». Les colonnes correspondantes (c'est-à-dire celles dont la longueur d'onde et/ou gamme de longueurs d'onde sont identiques) seront ensuite éliminées de la base de données spectrales A. Il faut noter que dans les exemples ci-dessus, les tableaux A et B constituent des représentations qui n'ont pas de véritables relations entre elles ; il faut également noter que les tableaux A et B ont été tronqués de manière à en donner une représentation visuelle ; en réalité, les dits tableaux comprennent une multitude de colonnes représentant les longueurs d'onde et/ou gammes de longueurs d'onde extraites du spectre correspondant comme détaillé plus avant dans la description.

Un exemple de représentation de la banque de données spectrales améliorée A' est donc illustré dans la figure 4.

Une caractéristique essentielle de ce procédé optionnel selon la présente invention consiste en ce que l'établissement de la banque de données spectrales améliorée A' n'a pas nécessité à ce stade de faire référence et/ou la moindre corrélation avec les propriétés chimiques et/ou physico-chimiques des étalons. En effet, cette deuxième étape en est totalement indépendante.

Une troisième étape consécutive préférée de ce procédé optionnel selon la présente invention consiste en l'élargissement proprement dit de la banque de données spectrales A (ou de la banque de données spectrales améliorée A'). Cette étape consiste à générer des étalons synthétiques (également appelés « intergermes » [« IG »]) à partir des étalons disponibles et de leurs valeurs de grandeurs spectrales. Par exemple, pour générer ces IG on peut effectuer des combinaisons de plusieurs étalons disponibles de la première étape ci-dessus et peupler la banque de données spectrales A (ou la banque de données spectrales améliorée A') au moyen des dites combinaisons. Ces combinaisons peuvent se faire de manière aléatoire ou de manière orientée tel que décrit plus avant dans le texte. Les dites combinaisons peuvent consister en toute sorte de traitement mathématique appliqué aux valeurs de grandeurs spectrales des étalons G. Selon un mode d'exécution préféré de la présente invention la dite combinaison consiste en un barycentre des valeurs de grandeurs spectrales (« VGS ») d'au moins deux étalons. On pourra par exemple effectuer ces combinaisons entre deux, trois ou un nombre supérieur des étalons disponibles de départ, de préférence entre tous les étalons disponibles de départ.

Un exemple de formule correspondante pour la génération d'un étalon synthétique (IG) à partir des étalons G (auquel correspondent les VGS) est

$$[\Sigma \, Ri \, x \, VGSi] / [\Sigma \, Ri]$$

pour i étant un nombre entier allant de 1 au nombre d'étalons G choisis pour cette combinaison et R étant un nombre réel tel que

$$[\Sigma \text{ Ri}] > 0,$$

et

$$\|[\Sigma \text{ R*i}]\| / [\Sigma \text{ Ri}] \quad < \quad 0.3 \quad, \text{de préférence} < 0.15,$$

Et avec R* représentant uniquement les nombres réels négatifs. Cette dernière formule peut aussi s'énoncer comme étant la valeur absolue de la somme des réels négatifs divisée par la somme de tous les réels.

Selon un mode d'exécution préféré de la présente invention, au moins un des Ri est un réel négatif (R*).

En procédant de la sorte, cela permet donc d'élargir au moyen d'étalons synthétiques (également appelés « intergermes » ou « IG ») la banque de données spectrales A (ou la banque de données spectrales améliorée A') et donc d'obtenir une banque de données spectrales élargie E.

Selon un mode d'exécution préféré de la présente invention, lorsque le nombre d'étalons de la banque de données spectrales A (ou A') vaut « N », le nombre d'intergermes IG est au moins supérieur à 1,5 N, de préférence supérieur à 2 N, plus préférentiellement supérieur à 5 N, voire supérieur à 10 N.

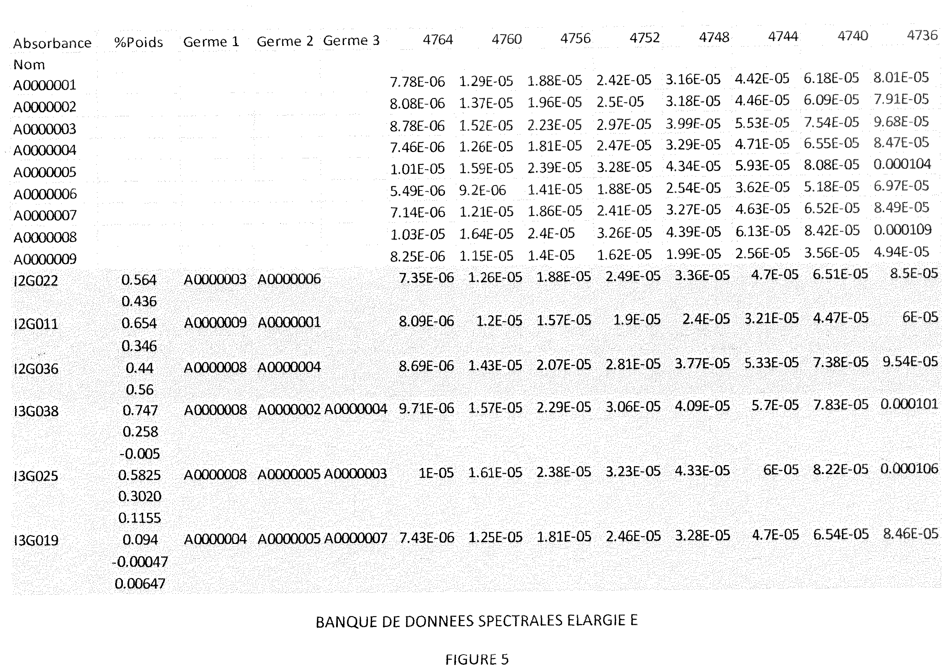

Un exemple de représentation de la banque de données spectrales élargie E est illustré dans la figure 5 par un tableau. On peut y voir que des étalons synthétiques (ou intergermes « IG ») ont été générés par combinaisons mathématiques et que les valeurs de grandeurs spectrales correspondantes ont été enregistrées dans le tableau E. On peut observer à titre d'exemple dans le tableau E (Figure 5) :

- six intergermes « IG » (I2G022, I2G011, I2G036, I3G038, I3G025 et I3G019;
- dans les colonnes 3 à 5, les germes utilisés pour générer chacun des dits intergermes ;
- dans la colonne 2, la pondération appliquée aux germes sélectionnés pour le calcul des VGS des intergermes (par exemple, pour le calcul de l'intergerme I2G036, on a appliqué une pondération de (0,44 fois le germe A0000008 + 0,56 fois le germe A0000004)).

Une caractéristique essentielle de ce procédé optionnel selon la présente invention consiste en ce que l'établissement de la banque de données spectrales élargie E n'a pas nécessité à ce stade de faire référence et/ou la moindre corrélation avec les propriétés chimiques et/ou physico-chimiques des étalons. En effet, cette étape d'élargissement en est totalement indépendante.

Une quatrième étape additionnelle optionnelle et préférée selon la présente invention consiste ensuite en un élargissement supplémentaire de la banque de données spectrales A ou de la banque de données spectrales élargie E au moyen d'un autre type d'étalons synthétiques que nous appellerons « extragermes » (« EG »). Cette étape peut s'avérer particulièrement pertinente lorsque le produit à analyser contient une pluralité de composés chimiques, par exemple le pétrole.

Elle consiste dans une première séquence à enregistrer les données spectrales d'au moins un spectre correspondant à un (ou plusieurs) des composés chimiques des gaz et/ou pétroles concernés (aussi appelé « Pôle(s) »). Ensuite, dans une seconde séquence, on procède à un élargissement additionnel de la banque de données spectrales en utilisant le(s) dit Pôle(s) et en les combinant avec les germes « G » (on effectue donc une combinaison de leurs valeurs de grandeur spectrale VGS).

Cette seconde séquence consiste à générer des étalons synthétiques (également appelés « extragermes » [« EG »]) à partir du/des Pôle(s) et des étalons disponibles et de leurs valeurs de grandeurs spectrales. Par exemple, pour générer ces EG on peut effectuer des combinaisons de(s) Pôle(s) et de plusieurs étalons disponibles de la première étape ci-dessus et peupler la banque de données spectrales A et/ou E au moyen des dites combinaisons. Ces combinaisons peuvent se faire de manière aléatoire ou de manière orientée tel que décrit plus avant dans le texte. Les dites combinaisons peuvent consister en toute sorte de traitement mathématique appliqué aux valeurs de grandeurs spectrales des étalons G et du/des Pôle(s). Selon un mode d'exécution préféré de la présente invention la dite combinaison consiste en un barycentre des valeurs de grandeurs spectrales (« VGS ») des étalons G sélectionnés et du/des Pôle(s). On pourra par exemple effectuer ces combinaisons entre au moins un Pôle et un, deux, trois ou un nombre supérieur des étalons disponibles de départ, de préférence avec tous les étalons disponibles de départ. Ces combinaisons s'effectueront de préférence avec tous les Pôles disponibles, de préférence avec tous les Pôles correspondant à tous les composés chimiques constituant le produit analysé.

Un exemple de formule correspondante pour la génération d'un étalon synthétique de type EG à partir de Pôle(s) et d'étalons G (auxquels correspondent les VGS) est

$$[\Sigma \, Ri \, x \, VGSi + \Sigma \, Rj \, x \, VGSj] \, / \, [\Sigma \, Ri + \Sigma \, Rj]$$

pour i étant un nombre entier allant de 1 au nombre d'étalons G choisis pour cette combinaison, j étant un nombre entier allant de 1 au nombre de Pôle(s) choisis pour cette combinaison et R étant un nombre réel tel que

$$[\Sigma \, Ri + \Sigma \, Rj] > 0,$$

et

$$|[\Sigma \, R*i]| \, / \, [\Sigma \, Ri + \Sigma \, Rj] \; < 0.3 \; , \text{ de préférence} < \; 0.15, \qquad (I)$$

avec R* représentant uniquement les nombres réels négatifs,
et, de préférence, chaque Rj doit être tel que le rapport
Rj / [$\Sigma$ Ri + $\Sigma$ Rj] soit toujours compris entre l'opposé de la teneur minimale et la teneur maximale en pourcentage en poids du Pôles j dans les gaz et/ou pétroles.

La formule (I) ci-dessus peut aussi s'énoncer comme étant la valeur absolue de la somme des réels négatifs « i » divisée par la somme de tous les réels. Selon un mode d'exécution préféré de la présente invention, au moins un des Ri est un réel négatif (R*).

En procédant de la sorte, cela permet donc d'élargir au moyen d'étalons synthétiques « EG » (« extragermes ») la banque de données spectrales A et/ou E et donc d'obtenir une banque de données spectrales élargie EE. De manière optionnelle, les dits Pôles et leurs VGS peuvent aussi être intégrés dans la banque de données spectrales EE mais ceci ne constitue pas un mode d'exécution préféré selon la présente invention.

Selon un mode d'exécution préféré de la présente invention, lorsque le nombre d'étalons de la banque de données spectrales A (ou A') vaut « N » et le nombre de « Pôles » vaut « M » , le nombre d'extragermes « EG » est au moins supérieur à NxM , de préférence supérieur à 1,5 NxM, de préférence supérieur à 2 NxM.

Selon un mode d'exécution de la présente invention, le nombre de pôles est inférieur à 15, par exemple inférieur à 10.

Selon un mode d'exécution de la présente invention, le nombre de pôles est inférieur à 0,2 fois le nombre d'étalons, par exemple inférieur à 0,1 fois le nombre d'étalons.

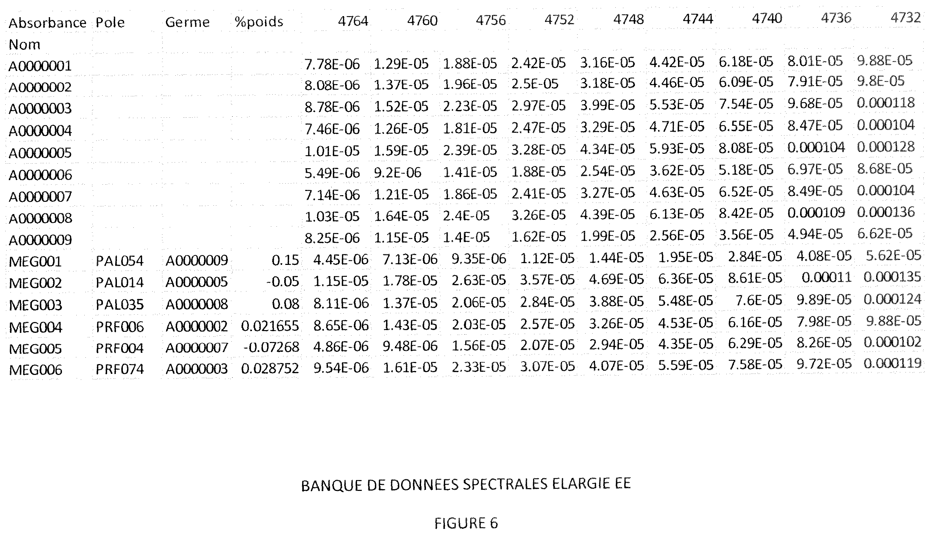

Un exemple de représentation de la banque de données spectrales élargie EE est illustré dans la figure 6 par le tableau EE. On peut y voir que les « Pôles » ainsi que la génération des étalons synthétiques « EG » (extragermes) par combinaisons mathématiques et les valeurs de grandeurs spectrales correspondantes ont été enregistrées dans le tableau. On peut observer à titre d'exemple dans le tableau EE (Figure 6) :

- six extragermes « EG » (MEG001 à MEG006);
- dans la colonne 2 (« Pôle »), la référence des pôles utilisés (par exemple, le Pôle PAL054 est un type particulier d'alkylat utilisé dans la composition d'essences constituant les étalons de la banque de données) ;
- dans la colonne 3, la référence du germe utilisé pour générer chacun des dits extragermes ;
- dans la colonne 4, la pondération appliquée aux Pôles (X)- la pondération appliquée aux germes étant donc de (1-X). Par exemple, pour le calcul de l'extragerme MEG001, on a appliqué une pondération de (0,15 fois le Pôle PAL054 + 0,85 fois le germe A0000009).

Une caractéristique essentielle de ce procédé optionnel selon la présente invention consiste en ce que l'établissement de la banque de données spectrales élargie EE n'a pas nécessité à ce stade de faire référence et/ou la moindre corrélation avec les propriétés chimiques et/ou physico-chimiques des étalons. En effet, cette étape d'élargissement en est totalement indépendante.

Une cinquième étape additionnelle optionnelle et préférée selon la présente invention consiste également en un élargissement supplémentaire de la banque de données spectrales élargie E et/ou EE au moyen d'un autre type d'étalons synthétiques que nous appellerons « extragermes' » (« EG' »). Cette étape est à nouveau particulièrement pertinente lorsque le produit à analyser contient une pluralité de composés chimiques, par exemple les pétroles.

Elle consiste dans une première séquence à enregistrer les données spectrales d'au moins un spectre correspondant à un (ou plusieurs) des composés chimiques du produit (aussi appelé « Pôle(s) »).

Ensuite, dans une seconde séquence, on procède à un élargissement additionnel de la banque de données spectrales E ou EE en utilisant le(s) dit(s) Pôle(s) et en les combinant avec les intergermes « IG » (combinaison de leurs VGS).

Cette seconde séquence consiste à générer des étalons synthétiques (également appelés « extragermes' » [« EG' »]) à partir du/des Pôle(s) et des étalons « intergermes » « IG » (et optionnellement des germes « G ») et de leurs valeurs de grandeurs spectrales. Par exemple, pour générer ces EG' on peut effectuer des combinaisons de(s) Pôle(s) et de plusieurs intergermes « IG » de la troisième étape ci-dessus (et optionnellement de germes « G » de la première étape) et peupler la banque de données spectrales E et/ou EE au moyen des dites combinaisons.

Ces combinaisons peuvent se faire de manière aléatoire ou de manière orientée tel que décrit plus avant dans le texte. Les dites combinaisons peuvent consister en toute sorte de traitement mathématique appliqué aux valeurs de grandeurs spectrales des étalons synthétiques (intergermes) « IG » et du/des Pôle(s) (et optionnellement des germes « G »).

Selon un mode d'exécution préféré de la présente invention la dite combinaison consiste en un barycentre des valeurs de grandeurs spectrales (« VGS ») des intergermes IG et du/des Pôle(s)) (et optionnellement des germes « G »). On pourra par exemple effectuer ces combinaisons entre au moins un Pôle et un, deux, trois ou un nombre supérieur des « IG » de la troisième étape, de préférence avec tous les « IG » ; et optionnellement avec au moins un des germes « G », de préférence avec tous les germes « G ». Ces combinaisons s'effectueront de préférence avec tous les Pôles disponibles, de préférence avec tous les Pôles correspondant à tous les composés chimiques constituant le produit analysé.

Un exemple de formule correspondante pour la génération d'un étalon synthétique de type EG' à partir de Pôle(s) et d'étalons synthétiques IG (auxquels correspondent les VGS) est
[$\sum$ Ri x VGSi + $\sum$ Rj x VGSj + $\sum$ Rk x VGSk] / [$\sum$ Ri + $\sum$ Rj + $\sum$ Rk] pour k étant un nombre entier allant de 1 au nombre d'étalons synthétiques IG choisis pour cette combinaison, i étant un nombre entier allant de 0 (de préférence 1) au nombre d'étalons G choisis pour cette combinaison, j étant un nombre entier allant de 1 au nombre de Pôle(s) choisis pour cette combinaison et R étant un nombre réel tel que

$$[\sum \text{Ri} + \sum \text{Rj} + \sum \text{Rk}] > 0,$$

et

$$\left|[\sum \text{R*i}] + [\sum \text{R*k}]\right| / [\sum \text{Ri} + \sum \text{Rj} + \sum \text{Rk}] \quad < \quad 0.3 \text{ , de préférence} < 0.15, \qquad (\text{II})$$

avec Rk étant de préférence toujours positif,
avec R* représentant uniquement les nombres réels négatifs,
ET de préférence chaque Rj doit être tel que le rapport
Rj / [$\sum$ Ri + $\sum$ Rj + $\sum$ Rk] soit toujours compris entre l'opposé de la teneur minimale et la teneur maximale en pourcentage en poids du Pôles j dans le produit analysé. La formule (II) ci-dessus peut aussi s'énoncer comme étant la valeur absolue de la somme des réels négatifs « i » divisée par la somme de tous les réels. Selon un mode d'exécution préféré de la présente invention, au moins un des Ri est un réel négatif (R*). En procédant de la sorte, cela permet donc d'élargir au moyen de d'étalons synthétiques « EG' » (« extragermes' ») la banque de données spectrales E et/ou EE et donc d'obtenir une banque de données spectrales élargie EEI. De manière optionnelle, les dits Pôles et leurs VGS peuvent aussi être intégrés dans la banque de données spectrales E mais ceci ne constitue pas un mode d'exécution préféré selon la présente invention.

Selon un mode d'exécution préféré de la présente invention, lorsque le nombre d'étalons synthétiques IG de la banque de données spectrales E vaut « Z » et le nombre de « Pôles » vaut « M » , le nombre d'extragermes' « EG' » est au moins supérieur à ZxM , de préférence supérieur à 1,5 ZxM, de préférence supérieur à 2 ZxM. Selon un autre mode d'exécution préféré de la présente invention, lorsque le nombre d'étalons synthétiques IG de la banque de données spectrales E vaut « Z », le nombre de germes G vaut N et le nombre de « Pôles » vaut « M », le nombre d'extragermes' « EG' » est au moins supérieur à ZxMxN , de préférence supérieur à 1,5 ZxMxN, de préférence supérieur à 2 ZxMxN.

Selon un mode d'exécution de la présente invention, le nombre de pôles est inférieur à 15, par exemple inférieur à 10.

Selon un mode d'exécution de la présente invention, le nombre de pôles est inférieur à 0,2 fois le nombre d'étalons, par exemple inférieur à 0,1 fois le nombre d'étalons.

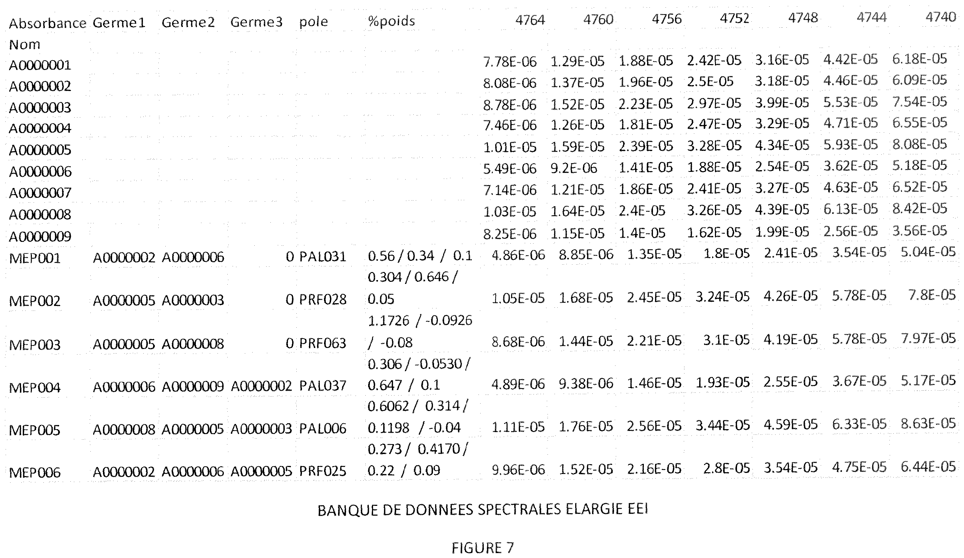

Un exemple de représentation de la banque de données spectrales élargie EEI est illustré dans la figure 7 par un tableau. On peut y voir les « Pôles » ainsi que la génération des étalons synthétiques « EG' » (extragermes') par combinaisons mathématiques et que les valeurs de grandeurs spectrales correspondantes ont été enregistrées dans le tableau. On peut observer à titre d'exemple dans le tableau EEI (Figure 7) :

- six extragermes' « EG' » (MEP001 à MEP006);
- dans la colonne 5 (« Pôle »), la référence des pôles utilisés (par exemple, le Pôle PAL037 est un type particulier d'alkylat utilisé dans la composition d'essences constituant les étalons de la banque de données) ;
- dans les colonnes 2 à 4, la référence des intergermes (combinaisons de germes) utilisé pour générer chacun des dits extragermes ;
- dans la colonne 6, la pondération appliquée. Par exemple, pour le calcul de l'extragerme MEP004, on a appliqué une pondération de [0,9 fois un intergerme (correspondant à 0,306 fois le germe A00000061 - 0,0530 fois le germe A0000009 + 0,647 fois le germe A0000002) + 0,1 fois le pôle PAL037].

Une caractéristique essentielle de ce procédé optionnel selon la présente invention consiste en ce que l'établissement de la banque de données spectrales élargie EEI n'a pas nécessité à ce stade de faire référence et/ou la moindre corrélation avec les propriétés chimiques et/ou physico-chimiques des étalons. En effet, cette étape d'élargissement en est totalement indépendante.

La présente invention concerne donc également une méthode de génération et d'optimisation d'une banque de données spectrales pouvant servir dans un procédé de caractérisation de l'origine des gaz et/ou pétroles (et/ou de leurs constituants) de différentes origines dans un mélange de ces derniers par analyse spectrale topologique à partir d'un nombre limité d'étalons disponibles,
méthode consistant dans une première étape

- à effectuer la même analyse spectrale sur les dits étalons, et
- à constituer à partir des spectres obtenus une banque de données spectrales A à plusieurs longueurs d'onde et/ou gammes de longueurs d'onde,

caractérisée dans une seconde étape optionnelle en ce qu'on élimine de la banque de données spectrales A les longueurs d'onde et/ou gammes de longueurs d'onde « polluantes » de la dite banque de données spectrales A, seconde étape consistant

1. à répéter au moins deux fois, de préférence au moins trois fois, plus préférentiellement au moins cinq fois la même analyse spectrale que celle effectuée lors de la première étape, et ceci sur au moins un des étalons disponibles, de préférence sur au moins deux ou même sur la totalité des dits étalons disponibles ;
2. à construire une banque de données spectrales B à partir des mesures effectuées au point 1 ci-dessus ;
3. à calculer pour chaque étalon sélectionné au point 1 ci-dessus et pour chaque longueur d'onde et/ou gamme de longueur d'onde (de la banque de données spectrales A) les écarts-type ($\sigma$) des mesures enregistrées dans la banque de données B ;
4. à identifier dans la banque de données B les longueurs d'onde et/ou gamme de longueur d'onde pour lesquelles l'écart-type est supérieur à une valeur prédéterminée ; et
5. à supprimer de la banque de données spectrales A les mesures correspondant aux longueurs d'onde identifiées au point 4 ci-dessus et obtenir ainsi une banque de données spectrales améliorée A',

et également caractérisée par une troisième étape consécutive préférée qui consiste en l'élargissement de la banque de données spectrales A (ou de la banque de données spectrales améliorée A'), étape consistant à effectuer des combinaisons de plusieurs étalons de la première étape et à peupler la banque de données spectrales A (ou la banque de données spectrales améliorée A') au moyen des dites combinaisons (appelées étalons synthétiques ou intergermes « IG ») et obtenir ainsi une banque de données spectrales élargie E,
et également caractérisée par une quatrième étape consécutive optionnelle qui consiste en l'élargissement de la banque de données spectrales E, étape consistant dans une première séquence à rajouter à la banque de données spectrales élargie E au moins un spectre correspondant à au moins un des composés chimiques (ou plusieurs) des gaz et/ou pétroles (et/ou de leurs constituants) de différentes origines (aussi appelé « Pôle(s) ») et dans une seconde séquence à effectuer des combinaisons mathématiques du(des) dit(s) Pôle(s) avec au moins un étalon G de la première étape et/ou au moins un des étalons IG de la troisième étape et à peupler la banque de données spectrales E au moyen des dites combinaisons (appelées respectivement soit étalons synthétiques extragermes « EG », soit étalons synthétiques extragermes' « EG' ») et obtenir ainsi une banque de données spectrales élargie EE (ou EEI).

Apres avoir constitué la banque de données spectrales (de préférence élargie conformément à la méthodologie développée ci-dessus) pour chacun des gaz et/ou pétroles et leurs mélange transporté, il est possible de mettre en évidence par comparaison entre les dites banques de données (en utilisant par exemple toute sorte d'analyse mathématique conventionnelle) un domaine spectral caractérisant qui discrimine entre elles les origines des dits gaz et/ou pétroles et ainsi déterminer les origines et les ratios respectifs des gaz et/ou pétroles dans le mélange final transporté à partir des banques de données spectrales élargies. On citera à titre illustratif d'analyse mathématique l'analyse spectrale topologique et/ou l'analyse par régression des moindres carrés partiels (PLS) ; pour permettre la caractérisation et la discrimination des origines (d'extraction) des gaz et/ou pétroles dans le mélange transporté.

Selon un mode d'exécution préféré de la présente invention, avant cette caractérisation, une étape intermédiaire additionnelle consiste à définir une méthode de discrimination efficace permettant de mettre en évidence des sous-groupes homogènes de produits qui obéissent préférentiellement aux mêmes types de liaisons propriétés-spectres par suite d'une forte analogie de structure moléculaire.

Les méthodes de discrimination peuvent être exclusivement basées sur des techniques d'analyse mathématiques (par exemple, des analyses factorielles et/ou des analyses en composantes principales). Bien que certaines de ces méthodes mathématiques puissent s'avérer utiles, la présente invention utilise de préférence encore au moins une étape empirique pour effectuer ce type de discrimination, étape empirique basée sur une analyse visuelle des spectres (par exemple des étalons et/ou des pôles susmentionnés) ; bien que ceci ne constitue pas un mode d'exécution préféré selon la présente invention, cette analyse visuelle pourrait également se faire sur les spectres reconstitués (à partir de leurs VGS calculées) des intergermes et/ou extragermes. Cette étape empirique permet ainsi de mettre en évidence des différences infimes entre les spectres en question, différences qui après vérification peuvent s'avérer synonymes de l'existence de sous-groupes homogènes de produits même si on pouvait penser à l'origine que l'ensemble de la population des produits était homogène. Cette technique de discrimination permet ainsi de mettre en évidence des différences entre les produits alors même que l'utilisateur final n'en avait pas encore la connaissance.

Pour rappel, une caractéristique essentielle du procédé optionnel d'établissement de la banque de données spectrales élargie selon un mode préféré d'exécution de l'invention susmentionnée est qu'il n'a pas été nécessaire de faire référence et/ou la moindre corrélation avec les propriétés chimiques et/ou physico-chimiques des étalons. Selon un mode d'exécution préféré de la présente invention, il en est exactement de même pour l'étape de discrimination décrite ici.

Ainsi, selon un mode d'exécution optionnel de la présente invention, l'étape de caractérisation et de discrimination consiste à définir, à partir de la banque de données spectrales (de préférence élargie), des agrégats (de préférence au moins deux agrégats), des espaces à n dimensions représentant les combinaisons des dits agrégats (de préférence des plans - ou espaces à deux dimensions - représentant des couples d'agrégats), et des boites spectrales correspondantes. Selon un mode d'exécution préféré de la présente invention, ces agrégats et/ou ces espaces à n dimensions représentant des combinaisons des dits agrégats et/ou ces boites spectrales définissent le domaine spectral caractérisant et discriminant des origines des dits gaz et/ou pétroles ce qui permet ainsi de déterminer les origines et les ratios respectifs des gaz et/ou pétroles dans le mélange final transporté.

Selon un mode d'exécution de la présente invention, la méthode de discrimination comprend également au moins deux caractéristiques préférées particulières :

1. le fait que la dite méthode implique une phase d'itération lors de laquelle on vérifie l'efficacité de la boite spectrale et donc la pertinence des agrégats sélectionnés ; et
2. le fait que les agrégats sont bâtis à partir d'au moins une analyse visuelle de l'allure des spectres qui permet ensuite de bâtir les équations des agrégats en fonction des valeurs de grandeurs spectrales VGS.

Les agrégats sont donc définis comme des fonctions mathématiques des valeurs de grandeurs spectrales de la banque de données spectrales élargie permettant de regrouper et/ou discriminer et/ou séparer des familles de produits (en l'occurrence des origines différentes des dits gaz et/ou pétroles) au sein de la banque de données spectrales élargie.

Ces agrégats peuvent donc être représentés de manière générique par la fonction Agg = f (VGSi).

Selon un mode d'exécution préféré de la présente invention, la dite fonction répond aux équations

de type $\dfrac{\sum_{k=1}^{n}\sum_{i=1}^{p} ai\, Wi^{\propto} Wk^{\beta}}{\sum_{i=1}^{q} ai\, Wi^{\propto}}$ ou de préférence de type $\dfrac{\sum_{i=1}^{p} ai\, Wi^{\propto}}{\sum_{i=1}^{q} ai\, Wi^{\propto}}$ dans lesquelles

- W représentent les valeurs de grandeurs spectrales VGS discriminantes,
- a sont des réels positifs,
- p et q représentent la sélection des VGS aux longueurs d'ondes et/ou gammes de longueurs d'onde pertinentes pour l'étape de discrimination, et
- $\propto$ et $\beta$ sont des exposants compris entre 1/3 et 3.

En ce qui concerne la phase d'itération lors de laquelle on vérifie l'efficacité de la boite spectrale et donc la pertinence des agrégats sélectionnés, il suffit de rajouter à la banque de données spectrales préétablie des colonnes représentant les équations des agrégats discriminants, de calculer la valeur des dits agrégats pour chacun des étalons et/ou intergermes et/ou extragermes et/ou pôles de la banque de données spectrales, d'en faire les représentations graphiques (de préférence dans des espaces à deux dimensions par paire d'agrégats), et de visualiser ainsi si la discrimination a bien conduit à la mise en évidence de sous-groupes homogènes de produits (en l'occurrence des origines différentes des dits gaz et/ou pétroles). Cette étape de discrimination permet donc de diviser la banque de données spectrales en plusieurs (au moins deux) familles distinctes (sous-groupes homogènes de produits ; en l'occurrence des origines différentes des dits gaz et/ou pétroles), de préférence au moins trois familles distinctes.

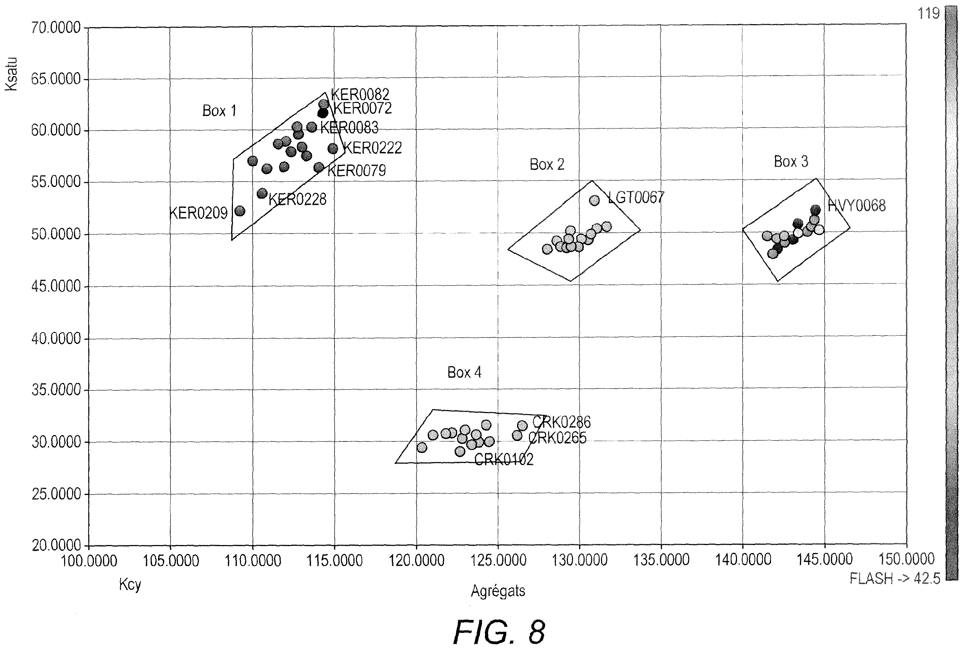

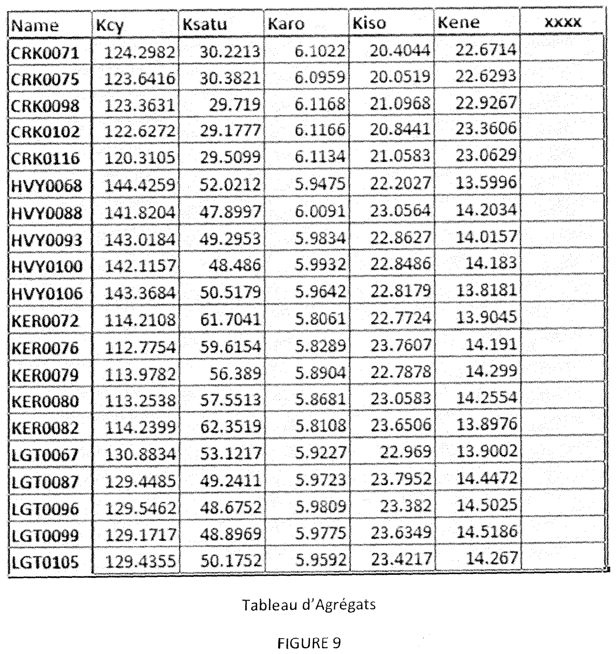

A titre d'exemple, les figures 8 et 9 représentent respectivement

- un graphique dont les axes abscisses/ordonnées correspondent à deux agrégats discriminants, et
- un tableau de valeurs correspondantes dont les colonnes représentent plusieurs agrégats discriminants dont les deux premiers ont servi à la constitution du graphique (figure 8).

Ces figures expliquent clairement comment on parvient à mettre en évidence plusieurs sous-groupes homogènes de produits (en l'occurrence des origines différentes des dits gaz et/ou pétroles); ce qui permet la sélection du domaine spectral caractérisant et discriminant.

La présente invention concerne donc également un procédé de caractérisation d'un produit (en l'occurrence des origines différentes des dits gaz et/ou pétroles) par analyse spectrale topologique.

La caractérisation d'un produit selon la présente invention peut consister en une détermination et/ou une prédiction de toute caractéristique chimique, physique ou physico-chimique du dit produit.

Selon un mode d'exécution particulier de la présente invention, la première étape a donc été caractérisée par l'établissement d'une banque de données spectrales, de préférence une banque de données spectrales élargies comme décrite dans la présente description.

Comme déjà indiqué ci-dessus, les représentations graphiques des banques de données (tableaux) dans les figures annexées constituent des vues tronquées car en réalité les dites banques de données comprennent une multitude de colonnes représentant les longueurs d'onde et/ou gammes de longueurs d'onde (ou à titre équivalent, les nombres d'onde ou gamme de nombres d'onde) extraites des spectres correspondants.

Selon un mode d'exécution de la présente invention, le nombre de longueurs d'onde choisi peut être de 2 à 1000, par exemple de 5 à 200 ou de 40 à 80.

Les longueurs d'onde choisies peuvent être à des intervalles réguliers tels que 1 à 50 nm ou tous les 10 à 50 nm ou tous les 15 à 35 nm ou tous les 1 à 5 nm ou tous les nanomètres ; ou elles peuvent être à des intervalles irréguliers, par exemple à des intervalles de 1 à 200 nm, par exemple de 1 à 100 ou de 1 à 50 en particulier de 2 à 50 ou de 4 à 50 ou de 10 à 60 nm, qui peuvent être choisis ou aléatoires en raison d'une variation de la forme de la courbe spectrale à cette longueur d'onde par exemple, un pic, une vallée ou un épaulement ou bien choisis avec des critères chimiques ou statistiques tels que l'analyse factorielle. Les longueurs d'onde peuvent être dans la région des 625 à 2600 nm, par exemple de 800 à 2600 nm, en particulier de 1500 à 2600 ou de 2000 à 2550 nm. Les nombres d'ondes peuvent être dans la région des 16000 à 3840 cm-1, par exemple de 12500 à 3840 cm-1 en particulier de 6660 à 3840 ou de 5000 à 3900 cm-1; les fréquences correspondantes en Hertz peuvent être obtenues en multipliant ces longueurs d'onde par 3x10(exp)10 cm/s.

Avant de pouvoir déterminer et/ou prédire une propriété d'un échantillon (en l'occurrence d'un mélange de gaz et/ou pétroles d'origines différentes), il faut bien évidemment mesurer les valeurs de la dite propriété pour les étalons et, optionnellement, pour les pôles. Ainsi, selon un mode d'exécution de la présente invention, les propriétés chimiques, physiques et/ou physico-chimiques des étalons (et optionnellement des pôles) sont déterminées au moyen de techniques d'analyses conventionnelles. A titre d'exemple non limitatif des techniques d'analyse conventionnelle, on citera la chromatographie en phase gazeuse pour les compositions chimiques. Bien qu'il aille de soi que les étalons soient choisis pour couvrir la gamme dans laquelle on doit utiliser la méthode, la présente invention permet dans un mode d'exécution préféré de travailler avec un nombre limité d'étalons grâce à la méthodologie d'élargissement de la banque de données spectrales susmentionnée.

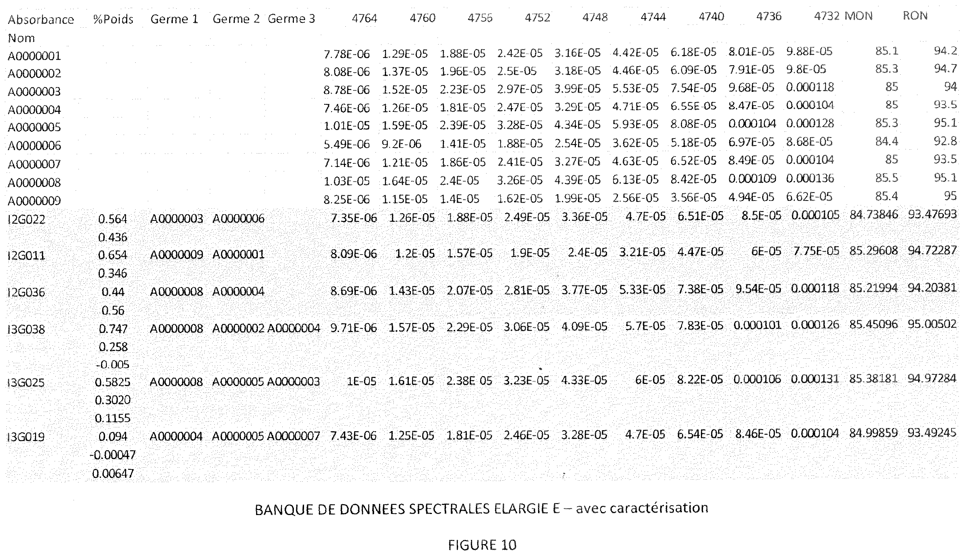

Ainsi, dans un mode d'exécution préféré de la présente invention, on rajoute à la banque de données spectrales les valeurs des propriétés désirées mesurées pour les dits étalons (et optionnellement des pôles); lorsque la banque de données spectrales est élargie, on calcule ensuite les valeurs des dites propriétés pour les étalons synthétiques intergermes (et optionnellement pour les extragermes) à partir des formules ayant servi à générer ces étalons synthétiques ; ce calcul se fait simplement en remplaçant les valeurs de grandeurs spectrales VGS par les valeurs mesurées des dites propriétés des étalons (et optionnellement des pôles) utilisés dans les formules (et optionnellement, pour les extragermes, par les valeurs déjà calculées pour les intergermes). On aboutit ainsi à une banque de données spectrales constituée d'un nombre de points (étalons et optionnellement intergermes, pôles et extragermes) auxquels sont associés les propriétés désirées (mesurées et calculées). Un exemple de réalisation (vue tronquée) est donné dans la figure 10.

Il s'agit à titre d'illustration d'une banque de données spectrales élargie E constituée d'étalons (A) et d'intergermes (IG). Le tableau a été complété au moyen des caractéristiques des gaz et/ou pétroles recherchées, à savoir les valeurs RON et MON (l'indice d'octane de recherche (RON) et l'indice d'octane de moteur (MON)). Ces caractéristiques ont donc été mesurées pour les étalons et calculées pour les intergermes.

Dans la description de EP0742900, on compare ensuite les signaux, par exemple les absorptions (ou leurs dérivées) pour l'échantillon inconnu, avec des signaux, par exemple les absorptions (ou leurs dérivées) à la même longueur d'onde des étalons, et on choisit les étalons ayant les différences les plus petites. Ensuite, on fait la moyenne des propriétés de ces étalons choisis pour déterminer la propriété de l'échantillon inconnu. On reconstitue donc un spectre calculé du produit cible auquel correspond la caractéristique (propriété) ainsi calculée.

Selon un mode d'exécution préféré de la présente invention, cette comparaison de signaux ne s'effectue donc pas sur l'entièreté de la banque de données spectrales mais uniquement sur la partie de banque de données spectrales représentative du sous-groupe homogène auquel l'échantillon appartient. C'est en utilisant de préférence la méthode de discrimination susmentionnée (agrégats discriminants) que cette partie de banque de données spectrales est définie.

Ensuite, on compare les signaux, par exemple les absorptions (ou leurs dérivées ou toute autre valeur de grandeur spectrale) pour l'échantillon inconnu (échantillon prélevé de préférence on-line dans la conduite de transport des gaz et/ou pétroles en mélange), avec les mêmes signaux et à la même longueur d'onde des étalons et/ou intergermes et/ou extragermes et/ou pôles appartenant au même sous-groupe homogène, et on choisit dans la banque de données spectrales les étalons et/ou intergermes et/ou extragermes et/ou pôles ayant les différences les plus petites.

Quelle que soit la méthode utilisée, nous appellerons par la suite les points proches du produit cible les points « proches voisins ». Ensuite, on peut par exemple faire la moyenne des propriétés de ces étalons et/ou intergermes et/ou extragermes et/ou pôles choisis pour déterminer la caractéristique recherchée (propriété) de l'échantillon inconnu.

Selon un mode d'exécution particulier de la présente invention, les proches voisins choisis sont ceux ayant les plus petites valeurs moyennes de la différence absolue à chaque longueur d'onde i entre la valeur de grandeur spectrale (représentée à titre d'exemple par l'absorbance ou une dérivée de celle-ci) $W_{ix}$ pour le produit cible (échantillon / produit inconnu) et le signal correspondant $W_{im}$ pour le proche voisin. Les moyennes peuvent se rapporter par exemple à la valeur moyenne de $W_{ix}-W_{im}$ (quel que soit son signe, à savoir une différence absolue), ou de $(W_{ix}-W_{im})exp2$. Pour chaque proche voisin dans la banque de données spectrales pour le type de produit en question, on trouve la différence moyenne telle que décrite et on choisit le proche voisin ayant les différences moyennes les plus petites, à savoir au moins 1 mais de préférence 2, jusqu'à 1000 des plus petites par exemple 2 à 100 ou 2 à 20 mais en particulier 2 à 10 et surtout 2 à 6 des plus petites. Cette sélection des plus proches voisins peut être effectuée selon toute méthode connue, par exemple on peut utiliser de manière avantageuse les méthodes décrites dans la description du brevet EP0742900 (par exemple en déterminant l'indice de proximité).

Selon un mode d'exécution particulier de la présente invention, le nombre de proches voisins peut être égal à 1, de préférence supérieur ou égal à 2, encore de préférence supérieur ou égal à 3.

Selon un mode d'exécution de la présente invention, le nombre de proches voisins est inférieur ou égal à 50, par exemple inférieur ou égal à 20, voir même 10.

Comme indiqué préalablement, à partir du moment où on a sélectionné les points « proches voisins », on peut facilement faire la moyenne des propriétés de ces proches voisins sélectionnés (étalons et/ou intergermes et/ou extragermes et/ou pôles) pour déterminer la propriété de l'échantillon inconnu (le produit cible). On reconstitue donc un spectre calculé du produit cible auquel correspond la caractéristique (propriété) ainsi calculée.

Toutefois, et ceci constitue un mode préféré d'exécution de la présente invention, la Demanderesse a découvert de manière inattendue une amélioration significative de la précision et robustesse de sa méthode lors de la détermination de la caractéristique recherchée (par exemple, une propriété) d'un produit cible lorsqu'on effectue une moyenne pondérée des propriétés de ces points « proches voisins » (qu'ils soient étalons et/ou intergermes et/ou extragermes et/ou pôles), la dite pondération étant une fonction inversement proportionnelle linéaire ou non-linéaire à la distance entre l'échantillon (« le produit cible ») et les points « proches voisins » sélectionnés ; cette pondération peut par exemple se représenter par la formule

$$\mathrm{POND} \;=\; \frac{\frac{1}{di^{\alpha}}}{\sum_{1}^{n}\frac{1}{di^{\alpha}}}$$

Avec $\alpha$ étant un nombre positif de préférence compris entre 0.5 et 1.5,
di étant la distance entre le produit cible et le proche voisin i, et
n étant le nombre total de proches voisins.

On applique donc une pondération de ce type aux propriétés mesurées (et optionnellement calculées) des « proches voisins » pour obtenir la propriété du produit cible.

On reconstitue donc un spectre calculé du produit cible auquel correspond la caractéristique (propriété) ainsi calculée.

En d'autres termes, le calcul de la caractéristique Z du produit cible se fait grâce aux caractéristiques correspondantes Zi des points proches voisins, tout en donnant aux caractéristiques des dits points proches voisins un poids d'autant plus important dans le dit calcul qu'ils sont plus proches du produit cible.

Ainsi, la présente invention concerne également une méthode de caractérisation d'un produit cible comprenant les étapes suivantes :

1. Constitution d'une banque de données spectrales comprenant des échantillons, leurs spectres et leurs caractéristiques mesurées (« CAR », par exemple la propriété « P »),
2. Analyse spectrale du produit cible et comparaison du spectre obtenu (Spectre PC) avec les données spectrales de la banque de données,
3. Identification des points « proches voisins » du produit cible, et
4. Calcul par topologie de la caractéristique du produit cible (CARpc/top , par exemple la propriété Ppc/top) en fonction des caractéristiques correspondantes des points proches voisins,

caractérisé en ce que le calcul de l'étape 4 est basé sur une pondération liée à l'inverse de la distance entre le produit cible et les points proches voisins.

On peut utiliser la méthode de l'invention pour déterminer plus d'une propriété P à la fois, par exemple au moins 2, en particulier de 1 à 30 par exemple 2 à 10 propriétés à la fois. On pourra bien évidemment utiliser des nombres différents d'étalons choisis pour chaque propriété.

Selon un autre mode préféré d'exécution de la présente invention, la Demanderesse a découvert une méthode alternative particulièrement efficace.

Cette méthode consiste à combiner l'une des méthodes topologiques de caractérisation du produit cible susmentionnées avec n'importe quel modèle mathématique différent des méthodes topologiques (de préférence un modèle régressionnel) et permettant de caractériser le produit cible à partir des valeurs de grandeurs spectrales VGS (pour la même propriété).

Cette méthode implique donc la constitution préalable d'un modèle mathématique qui permet de calculer les propriétés des produits en fonction des valeurs de grandeur spectrale (VGS) de la banque de données, de préférence un modèle régressionnel (de caractérisation de produit à partir de la banque de données spectrales préétablie) ; cette banque de données spectrales peut être soit la banque de données A susmentionnée ou de préférence la banque de données A', E, EE ou EEI, ou une sélection des dites banques. De préférence, cette banque de données sera la même que celle qui a servi pour la méthode topologique.

Cette méthode alternative pour caractériser un produit cible comprend les étapes suivantes :

1. Constitution d'une banque de données spectrales comprenant des échantillons, leurs spectres et leurs caractéristiques mesurées (« CAR », par exemple la propriété « P »),
2. Analyse spectrale du produit cible et comparaison du spectre obtenu (Spectre PC) avec les données spectrales de la banque de données,
3. Identification des points « proches voisins » du produit cible,
4. Calcul par topologie

    4.1. de la caractéristique du produit cible (CARpc/top , par exemple la propriété Ppc/top), et
    4.2. de son spectre ainsi calculé (spectre PCcalc),

5. Etablissement à partir de la banque de données spectrales d'un modèle mathématique permettant de calculer la caractéristique d'un produit à partir de la banque de données spectrales (CAR/mod, par exemple propriété P/mod),
6. Calcul de la caractérisation du produit cible PC en suivant la formule suivante CARpc = CARpc/top + [CARpc/mod - CARpccalc/mod]
avec

- CARpc étant la valeur calculée de la caractéristique du produit cible recherchée,
- CARpc/top étant la valeur calculée par topologie (points proches voisins) de la caractéristique du produit cible,
- CARpc/mod étant la valeur calculée par le modèle mathématique de la caractéristique du produit cible, et
- CARpccalc/mod étant la valeur calculée par le modèle mathématique de la caractéristique du produit cible calculé (au moyen des données spectrales obtenues au point 4.2).

La caractérisation d'un produit selon la présente invention peut donc consister en une détermination et/ou une prédiction de toute caractéristique chimique, physique ou physico-chimique du dit produit et/ou l'identification d'un type et/ou famille de produits.

On peut par exemple déterminer la présence de composés chimiques individuels au sein d'une composition ainsi que leurs concentrations ; on peut également déterminer toute sorte de propriétés utiles des dits gaz et/ou pétroles. Il doit être évident pour l'homme du métier que la présente invention permet des modes de réalisation sous de nombreuses autres formes spécifiques sans l'éloigner du domaine d'application de l'invention tel que revendiqué. Ainsi, les présents modes de réalisation doivent être considérés à titre d'illustration mais peuvent être modifiés dans le domaine défini par la portée des revendications jointes.

**Revendications**

1. Procédé pour déterminer la concentration et l'origine de gaz et/ou de pétroles dans une zone de mélange après mélange par transport des dits gaz et/ou pétroles provenant d'au moins deux origines différentes, le dit procédé comprenant

   - une analyse spectrale effectuée dans un domaine de longueurs d'ondes pour chaque gaz et/ou pétrole provenant des différentes origines,
   - une analyse spectrale effectuée dans le même domaine de longueurs d'ondes pour les gaz et/ou pétroles du mélange,
   - une étape de comparaison entre toutes les analyses spectrales,
   - et une étape de mise en évidence au moyen de cette comparaison entre les dites analyses spectrales d'un domaine spectral caractérisant, sans faire référence et/ou la moindre corrélation avec les propriétés chimiques et/ou physico-chimiques des dits gaz et/ou pétroles, qui permet de discriminer entre elles les différentes origines des dits gaz et/ou pétroles et de déterminer ainsi la concentration et l'origine de chacun des gaz et/ou pétroles dans le mélange,
   - le domaine spectral caractérisant étant déterminé par analyse spectrale topologique et les analyses spectrales étant effectuées dans le domaine proche infrarouge (« NIR »).

2. Procédé pour déterminer la concentration et l'origine de gaz et/ou de pétroles selon la revendication précédente **caractérisé en ce que** la dite origine constitue une origine d'extraction du dit gaz et/ou pétrole.

3. Procédé pour déterminer la concentration et l'origine de gaz et/ou de pétroles selon la revendication 2 **caractérisé en ce que** les origines d'extraction du dit gaz et/ou pétrole sont des puits et **en ce que** les analyses spectrales des gaz et/ou pétrole provenant de ces puits s'effectuent sur la plate-forme d'extraction, et/ou sur la tête de puits, et/ou à l'intérieur du puits d'extraction.

4. Procédé pour déterminer la concentration et l'origine de gaz et/ou de pétroles selon la revendication 3 **caractérisé en ce que** les analyses spectrales sont effectuées in situ en utilisant une fibre optique.

5. Procédé pour déterminer la concentration et l'origine de gaz et/ou de pétroles selon l'une quelconque des revendications précédentes **caractérisé en ce que** les analyses spectrales proviennent de spectres obtenus au moyen du même type de spectromètre.

6. Procédé pour déterminer la concentration et l'origine de gaz et/ou de pétroles selon l'une quelconque des revendications précédentes **caractérisé en ce que** l'étape de mise en évidence d'un domaine spectral caractérisant comprend une étape empirique basée sur une analyse visuelle des spectres.

7. Procédé pour déterminer la concentration et l'origine de gaz et/ou de pétroles selon l'une quelconque des revendications précédentes **caractérisé en ce que**

   - les analyses spectrales comportent la génération d'une banque de données spectrales comprenant les données spectrales d'échantillons représentatifs de chacun des gaz et/ou pétroles et une banque de données spectrales comprenant les données spectrales d'échantillons représentatifs du mélange,
   - l'étape de comparaison se fait par comparaison entre les banques de données au moyen de l'analyse spectrale topologique, et
   - cette étape de comparaison permet la mise en évidence du domaine spectral caractérisant et la détermination de la concentration et l'origine de chacun des gaz et/ou pétroles dans le mélange, sans faire référence et/ou la moindre corrélation avec les propriétés chimiques et/ou physico-chimiques des dits gaz et/ou pétroles.

8. Procédé pour déterminer la concentration et l'origine de gaz et/ou de pétroles selon la revendication 7 **caractérisé en ce que** le banque de données spectrales est élargie au moyen de combinaison de données spectrales représentatives de combinaison d'échantillons.

9. Procédé pour déterminer la concentration et l'origine de gaz et/ou de pétroles selon l'une quelconque des revendications 7 ou 8 **caractérisé en ce qu'**on élimine de la banque de données spectrales les longueurs d'onde et/ou gammes de longueurs d'onde polluantes en

a) répétant au moins deux fois, de préférence au moins trois fois, plus préférentiellement au moins cinq fois la même analyse spectrale sur au moins un des échantillons, de préférence sur au moins deux ou même sur la totalité des dits échantillons ;
b) en construisant une nouvelle banque de données spectrales à partir de ces mesures ;
c) en calculant pour chaque échantillon de l'étape a) ci-dessus les écarts-type ($\sigma$) des mesures enregistrées dans la nouvelle banque de données ;
d) en identifiant dans la nouvelle banque de données les longueurs d'onde et/ou gamme de longueur d'onde pour lesquelles l'écart-type est supérieur à une valeur prédéterminée ; et
e) en supprimant de la banque de données spectrales les mesures correspondant aux longueurs d'onde identifiées à l'étape d) ci-dessus.

10. Utilisation d'un procédé selon l'une quelconque des revendications précédentes afin de discriminer entre elles les différentes origines des dits gaz et/ou pétroles et de déterminer ainsi la concentration et l'origine de chacun des gaz et/ou pétroles dans le mélange.


**Patentansprüche**

1. Verfahren zum Bestimmen der Konzentration und Herkunft von Gasen und/oder Ölen in einer Mischzone nach Mischung durch Transport der Gase und/oder Öle, die mindestens zwei verschiedenen Herkünften entstammen, das Verfahren umfassend

- eine Spektralanalyse, die in einem Wellenlängenbereich für jedes Gas und/oder Öl, das den verschiedenen Herkünften entstammt, durchgeführt wird,
- eine Spektralanalyse, die in demselben Wellenlängenbereich für die Gase und/oder Öle des Gemischs durchgeführt wird,
- einen Schritt des Vergleichens zwischen allen Spektralanalysen,
- und einen Schritt des Nachweisens, mittels dieses Vergleichs zwischen den Spektralanalysen, eines charakterisierenden Spektralbereichs ohne Bezugnahme und/oder jegliche Korrelation mit den chemischen und/oder physikalisch-chemischen Eigenschaften der Gase und/oder Öle, der es gestattet, die verschiedenen Herkünfte der Gase und/oder Öle voneinander zu unterscheiden und so die Konzentration und Herkunft jedes der Gase und/oder Öle in dem Gemisch zu bestimmen,
- wobei der charakterisierende Spektralbereich durch topologische Spektralanalyse bestimmt wird und die Spektralanalysen im Nahinfrarotbereich ("NIR") durchgeführt werden.

2. Verfahren zum Bestimmen der Konzentration und Herkunft von Gasen und/oder Ölen nach dem vorhergehenden Anspruch, **dadurch gekennzeichnet, dass** die Herkunft eine Förderherkunft des Gases und/oder Öls bildet.

3. Verfahren zum Bestimmen der Konzentration und Herkunft von Gasen und/oder Ölen nach Anspruch 2, **dadurch gekennzeichnet, dass** die Förderherkünfte des Gases und/oder Öls Bohrlöcher sind, und dadurch, dass die Spektralanalysen der Gase und/oder Öle, die diesen Bohrlöchern entstammen, auf der Förderplattform und/oder auf dem Bohrlochkopf und/oder innerhalb des Förderbohrlochs erfolgen.

4. Verfahren zum Bestimmen der Konzentration und Herkunft von Gasen und/oder Ölen nach Anspruch 3, **dadurch gekennzeichnet, dass** die Spektralanalysen mithilfe eines Lichtwellenleiters in situ durchgeführt werden.

5. Verfahren zum Bestimmen der Konzentration und Herkunft von Gasen und/oder Ölen nach einem der vorhergehenden Ansprüche, **dadurch gekennzeichnet, dass** die Spektralanalysen von Spektren kommen, die mithilfe desselben Spektrometertyps erhalten wurden.

6. Verfahren zum Bestimmen der Konzentration und Herkunft von Gasen und/oder Ölen nach einem der vorhergehenden Ansprüche, **dadurch gekennzeichnet, dass** der Schritt des Nachweisens eines charakterisierenden Spektralbereichs einen empirischen Schritt umfasst, der auf einer visuellen Analyse der Spektren beruht.

7. Verfahren zum Bestimmen der Konzentration und Herkunft von Gasen und/oder Ölen nach einem der vorhergehenden Ansprüche, **dadurch gekennzeichnet, dass**

   - die Spektralanalysen das Erzeugen einer Spektraldatenbank umfassend die Spektraldaten von Proben, die für jedes der Gase und/oder Öle repräsentativ sind, und einer Spektraldatenbank umfassend die Spektraldaten von Proben, die für das Gemisch repräsentativ sind, aufweisen,
   - der Schritt des Vergleichens durch Vergleich zwischen den Datenbanken mittels der topologischen Spektralanalyse erfolgt, und
   - dieser Schritt des Vergleichens den Nachweis des charakterisierenden Spektralbereichs und die Bestimmung der Konzentration und Herkunft jedes der Gase und/oder Öle in dem Gemisch ohne Bezugnahme und/oder jegliche Korrelation mit den chemischen und/oder physikalisch-chemischen Eigenschaften der Gase und/oder Öle gestattet.

8. Verfahren zum Bestimmen der Konzentration und Herkunft von Gasen und/oder Ölen nach Anspruch 7, **dadurch gekennzeichnet, dass** die Spektraldatenbank durch Kombination von Spektraldaten erweitert wird, die für eine Probenkombination repräsentativ sind.

9. Verfahren zum Bestimmen der Konzentration und Herkunft von Gasen und/oder Ölen nach einem der Ansprüche 7 oder 8, **dadurch gekennzeichnet, dass** die verschmutzenden Wellenlängen und/oder Wellenlängenbereiche aus der Spektraldatenbank entfernt werden, indem

   a) dieselbe Spektralanalyse mindestens zweimal, vorzugsweise mindestens dreimal, besser noch mindestens fünfmal an mindestens einer der Proben, vorzugsweise an mindestens zwei oder sogar der Gesamtheit der Proben wiederholt wird;
   b) indem eine neue Spektraldatenbank ausgehend von diesen Messungen aufgebaut wird;
   c) indem für jede Probe des obigen Schritts a) die Standardabweichungen ($\sigma$) der Messungen, die in der neuen Datenbank gespeichert sind, berechnet werden;
   d) indem in der neuen Datenbank die Wellenlängen und/oder Wellenlängenbereiche identifiziert werden, für welche die Standardabweichung höher als ein vorbestimmter Wert ist; und
   e) indem die Messungen, die den Wellenlängen entsprechen, die beim obigen Schritt d) identifiziert wurden, aus der Spektraldatenbank gelöscht werden.

10. Nutzung eines Verfahrens nach einem der vorhergehenden Ansprüche, um die verschiedenen Herkünfte der Gase und/oder Öle voneinander zu unterscheiden und so die Konzentration und Herkunft jedes der Gase und/oder Öle in dem Gemisch zu bestimmen.

## Claims

1. Method for determining the concentration and the origin of gases and/or oils in a mixing zone after mixing by transportation of said gases and/or oils originating from at least two different origins, said method comprising

   - a spectral analysis performed in a range of wavelengths for each gas and/or oil originating from the different origins,
   - a spectral analysis performed in the same range of wavelengths for the gases and/or oils of the mixture,
   - a step of comparison between all the spectral analyses,
   - and a step of highlighting, by means of this comparison between said spectral analyses, of a characterizing spectral range, without reference to and/or the slightest correlation with the chemical and/or physicochemical properties of said gases and/or oils, which makes it possible to discriminate from one another the different origins of said gases and/or oils and thus to determine the concentration and the origin of each of the gases and/or oils in the mixture,

- the characterizing spectral range being determined by topological spectral analysis and the spectral analyses being performed in the near-infrared ("NIR") range.

2. Method for determining the concentration and the origin of gases and/or oils according to the preceding claim, **characterized in that** said origin constitutes an origin of extraction of said gas and/or oil.

3. Method for determining the concentration and the origin of gases and/or oils according to Claim 2, **characterized in that** the origins of extraction of said gas and/or oil are wells and **in that** the spectral analyses of the gases and/or oils originating from these wells are performed on the extraction platform, and/or on the well head, and/or inside the extraction well.

4. Method for determining the concentration and the origin of gases and/or oils according to Claim 3, **characterized in that** the spectral analyses are performed in situ by using an optical fibre.

5. Method for determining the concentration and the origin of gases and/or oils according to any one of the preceding claims, **characterized in that** the spectral analyses originate from spectra obtained by means of the same type of spectrometer.

6. Method for determining the concentration and the origin of gases and/or oils according to any one of the preceding claims, **characterized in that** the step of highlighting of a characterizing spectral range comprises an empirical step based on a visual analysis of the spectra.

7. Method for determining the concentration and the origin of gases and/or oils according to any one of the preceding claims, **characterized in that**

- the spectral analyses comprise the generation of a spectral databank comprising the spectral data of samples representative of each of the gases and/or oils and a spectral databank comprising the spectral data of samples representative of the mixture,
- the comparison step is done by comparison between the databank by means of the topological spectral analysis, and
- this comparison step makes it possible to highlight the characterizing spectral range and to determine the concentration and the origin of each of the gases and/or oils in the mixture, without reference to and/or the slightest correlation with the chemical and/or physicochemical properties of said gases and/or oils.

8. Method for determining the concentration and the origin of gases and/or oils according to Claim 7, **characterized in that** the spectral databank is expanded by means of combining spectral data representative of a combination of samples.

9. Method for determining the concentration and the origin of gases and/or oils according to either one of Claims 7 and 8, **characterized in that** the polluting wavelengths and/or ranges of wavelengths are eliminated from the spectral databank by

a) repeating at least twice, preferably at least three times, more preferentially at least five times, the same spectral analysis on at least one of the samples, preferably on at least two or even on all of said samples;
b) constructing a new spectral databank from these measurements;
c) calculating, for each sample of the above step a), the standard deviations ($\sigma$) of the measurements stored in the new databank;
d) identifying, in the new databank, the wavelengths and/or range of wavelengths for which the standard deviation is greater than a predetermined value; and
e) eliminating from the spectral databank the measurements corresponding to the wavelengths identified in the above step d).

10. Use of a method according to any one of the preceding claims in order to discriminate from one another the different origins of said gases and/or oils and thus to determine the concentration and the origin of each of the gases and/or oils in the mixture.

FIGURE 1

| Absorbances | Nombre d'onde (cm-1) | 4776 | 4772 | 4768 | 4764 | 4760 | 4756 | 4752 | 4748 | 4744 | 4740 | 4736 | 4732 | 4728 |
|---|---|---|---|---|---|---|---|---|---|---|---|---|---|---|
| Etalon | | | | | | | | | | | | | | |
| A0000001 | | 0.00000131 | 0.00000234 | 0.00000471 | 0.00000778 | 0.00001291 | 0.00001881 | 0.00002420 | 0.00003164 | 0.00004422 | 0.00006178 | 0.00008012 | 0.00009882 | 0.00011603 |
| A0000002 | | 0.00000154 | 0.00000300 | 0.00000484 | 0.00000808 | 0.00001365 | 0.00001962 | 0.00002497 | 0.00003183 | 0.00004456 | 0.00006093 | 0.00007915 | 0.00009801 | 0.00011606 |
| A0000003 | | 0.00000131 | 0.00000284 | 0.00000512 | 0.00000878 | 0.00001523 | 0.00002234 | 0.00002966 | 0.00003987 | 0.00005529 | 0.00007543 | 0.00009683 | 0.00011817 | 0.00013911 |
| A0000004 | | 0.00000028 | 0.00000173 | 0.00000375 | 0.00000746 | 0.00001259 | 0.00001808 | 0.00002468 | 0.00003287 | 0.00004706 | 0.00006552 | 0.00008471 | 0.00010404 | 0.00012267 |
| A0000005 | | 0.00000152 | 0.00000346 | 0.00000608 | 0.00001005 | 0.00001590 | 0.00002388 | 0.00003277 | 0.00004342 | 0.00005927 | 0.00008077 | 0.00010398 | 0.00012785 | 0.00014973 |
| A0000006 | | 0.00000025 | 0.00000137 | 0.00000276 | 0.00000549 | 0.00000920 | 0.00001410 | 0.00001884 | 0.00002541 | 0.00003622 | 0.00005183 | 0.00006968 | 0.00008685 | 0.00010424 |
| A0000007 | | 0.00000115 | 0.00000192 | 0.00000381 | 0.00000714 | 0.00001208 | 0.00001862 | 0.00002413 | 0.00003267 | 0.00004633 | 0.00006518 | 0.00008486 | 0.00010414 | 0.00012311 |
| A0000008 | | 0.00000145 | 0.00000286 | 0.00000597 | 0.00001025 | 0.00001641 | 0.00002401 | 0.00003255 | 0.00004393 | 0.00006125 | 0.00008424 | 0.00010897 | 0.00013581 | 0.00016053 |
| A0000009 | | 0.00000196 | 0.00000443 | 0.00000613 | 0.00000825 | 0.00001145 | 0.00001403 | 0.00001617 | 0.00001990 | 0.00002562 | 0.00003560 | 0.00004941 | 0.00006621 | 0.00008322 |

:

:

| Absorbances | Nombre d'onde (cm-1) | 4352 | 4348 | 4344 | 4340 | 4336 | 4332 | 4328 | 4324 | 4320 | 4316 | 4312 | 4308 | 4304 |
|---|---|---|---|---|---|---|---|---|---|---|---|---|---|---|
| Etalon | | | | | | | | | | | | | | |
| A0000001 | | 0.00966521 | 0.01052209 | 0.01158039 | 0.01273609 | 0.01366893 | 0.01401955 | 0.01384877 | 0.01353086 | 0.01333914 | 0.01318977 | 0.01295103 | 0.01250628 | 0.01190033 |
| A0000002 | | 0.00962570 | 0.01045172 | 0.01147713 | 0.01259841 | 0.01351549 | 0.01386926 | 0.01370155 | 0.01337238 | 0.01316494 | 0.01301322 | 0.01279028 | 0.01237890 | 0.01181515 |
| A0000003 | | 0.00971714 | 0.01051752 | 0.01151046 | 0.01260728 | 0.01350349 | 0.01385874 | 0.01371417 | 0.01342094 | 0.01323014 | 0.01306308 | 0.01280913 | 0.01238076 | 0.01181617 |
| A0000004 | | 0.00970203 | 0.01053299 | 0.01156151 | 0.01269044 | 0.01360349 | 0.01396508 | 0.01381938 | 0.01353130 | 0.01333327 | 0.01313686 | 0.01284140 | 0.01236973 | 0.01178167 |
| A0000005 | | 0.00983396 | 0.01059963 | 0.01155613 | 0.01260533 | 0.01346771 | 0.01381668 | 0.01367895 | 0.01339166 | 0.01320615 | 0.01304760 | 0.01281606 | 0.01240677 | 0.01184798 |
| A0000006 | | 0.00975312 | 0.01059458 | 0.01165282 | 0.01283536 | 0.01379368 | 0.01414591 | 0.01392945 | 0.01352795 | 0.01322576 | 0.01296382 | 0.01265066 | 0.01220577 | 0.01166779 |
| A0000007 | | 0.00971298 | 0.01053791 | 0.01157010 | 0.01271768 | 0.01364754 | 0.01400085 | 0.01381945 | 0.01347245 | 0.01322868 | 0.01300856 | 0.01271797 | 0.01227517 | 0.01172447 |
| A0000008 | | 0.00973713 | 0.01055416 | 0.01158105 | 0.01271146 | 0.01362481 | 0.01395885 | 0.01376642 | 0.01341727 | 0.01316979 | 0.01298435 | 0.01274264 | 0.01233134 | 0.01177974 |
| A0000009 | | 0.00973863 | 0.01054689 | 0.01155098 | 0.01265425 | 0.01355805 | 0.01391147 | 0.01373056 | 0.01336471 | 0.01310149 | 0.01290984 | 0.01267387 | 0.01227335 | 0.01172989 |

BANQUE DE DONNEES SPECTRALES A

FIGURE 2

EP 3 213 051 B1

EP 3 213 051 B1

| Longueur d'onde VGS | W1 | W2 | W3 | W4 | W5 | W6 | W7 | W8 | W9 |
|---|---|---|---|---|---|---|---|---|---|
| | 1.52455E-07 | 5.98E-06 | 5.43E-06 | 9.39E-06 | 1.38E-05 | 2.07E-05 | 2.62E-05 | 3.04E-05 | 4.14E-05 |
| | 1.12042E-07 | 6.81E-06 | 5.33E-06 | 9.54E-06 | 1.46E-05 | 2.15E-05 | 2.69E-05 | 3.11E-05 | 4.18E-05 |
| | 1.55755E-06 | 4.53E-06 | 6.07E-06 | 9.07E-06 | 1.45E-05 | 2.07E-05 | 2.6E-05 | 3.13E-05 | 4.12E-05 |
| | 4.15098E-08 | 6.65E-06 | 5.33E-06 | 9.63E-06 | 1.45E-05 | 2.09E-05 | 2.62E-05 | 3.12E-05 | 4.14E-05 |
| | 1.28445E-07 | 6.52E-06 | 5.19E-06 | 9.68E-06 | 1.46E-05 | 2.21E-05 | 2.7E-05 | 3.08E-05 | 4.2E-05 |
| | 1.52686E-06 | 4.24E-06 | 6.16E-06 | 8.66E-06 | 1.36E-05 | 1.97E-05 | 2.57E-05 | 3.07E-05 | 4.05E-05 |
| | 1.45709E-06 | 4.02E-06 | 6.06E-06 | 9.18E-06 | 1.47E-05 | 2.04E-05 | 2.63E-05 | 3.14E-05 | 4.1E-05 |
| | 1.8449E-06 | 4.52E-06 | 5.79E-06 | 8.99E-06 | 1.43E-05 | 2.02E-05 | 2.62E-05 | 3.16E-05 | 4.17E-05 |
| | 1.73044E-06 | 4.4E-06 | 6.12E-06 | 8.94E-06 | 1.42E-05 | 2.04E-05 | 2.63E-05 | 3.11E-05 | 4.07E-05 |
| | 1.11909E-06 | 3.85E-06 | 6.37E-06 | 9.31E-06 | 1.43E-05 | 2.08E-05 | 2.59E-05 | 3.07E-05 | 4.07E-05 |
| VGSm | 9.67038E-07 | 5.15E-06 | 5.79E-06 | 9.24E-06 | 1.43E-05 | 2.07E-05 | 2.63E-05 | 3.11E-05 | 4.12E-05 |
| $\sigma$ | 7.6254E-07 | 1.19E-06 | 4.27E-07 | 3.31E-07 | 3.79E-07 | 6.75E-07 | 4.21E-07 | 3.79E-07 | 4.99E-07 |
| $\frac{m}{\sigma} * 100$ | 78.85 | 23.09 | 7.38 | 3.58 | 2.65 | 3.26 | 1.60 | 1.22 | 1.21 |

BANQUE DE DONNEES SPECTRALES B

FIGURE 3

| Absorbances | Nombre d'onde (cm-1) | 4776 | 4772 | 4768 | 4764 | 4760 | 4756 | 4752 | 4748 | 4744 | 4740 | 4736 | 4732 | 4728 |
|---|---|---|---|---|---|---|---|---|---|---|---|---|---|---|
| Etalon | | | | | | | | | | | | | | |
| A0000001 | | 0.00000131 | 0.00000234 | 0.00000471 | 0.00000778 | 0.00001291 | 0.00001881 | 0.00002420 | 0.00003164 | 0.00004422 | 0.00006178 | 0.00008012 | 0.00009882 | 0.00011603 |
| A0000002 | | 0.00000154 | 0.00000300 | 0.00000484 | 0.00000808 | 0.00001365 | 0.00001962 | 0.00002497 | 0.00003183 | 0.00004456 | 0.00006093 | 0.00007915 | 0.00009801 | 0.00011606 |
| A0000003 | | 0.00000131 | 0.00000284 | 0.00000512 | 0.00000878 | 0.00001523 | 0.00002234 | 0.00002966 | 0.00003987 | 0.00005529 | 0.00007543 | 0.00009683 | 0.00011817 | 0.00013911 |
| A0000004 | | 0.00000028 | 0.00000173 | 0.00000375 | 0.00000746 | 0.00001259 | 0.00001808 | 0.00002468 | 0.00003287 | 0.00004706 | 0.00006552 | 0.00008471 | 0.00010404 | 0.00012267 |
| A0000005 | | 0.00000152 | 0.00000346 | 0.00000608 | 0.00001005 | 0.00001590 | 0.00002388 | 0.00003277 | 0.00004342 | 0.00005927 | 0.00008077 | 0.00010398 | 0.00012785 | 0.00014973 |
| A0000006 | | 0.00000025 | 0.00000137 | 0.00000276 | 0.00000549 | 0.00000920 | 0.00001410 | 0.00001884 | 0.00002541 | 0.00003622 | 0.00005183 | 0.00006968 | 0.00008685 | 0.00010424 |
| A0000007 | | 0.00000115 | 0.00000192 | 0.00000381 | 0.00000714 | 0.00001208 | 0.00001862 | 0.00002413 | 0.00003267 | 0.00004633 | 0.00006518 | 0.00008486 | 0.00010414 | 0.00012311 |
| A0000008 | | 0.00000145 | 0.00000286 | 0.00000597 | 0.00001025 | 0.00001641 | 0.00002401 | 0.00003255 | 0.00004393 | 0.00006125 | 0.00008424 | 0.00010897 | 0.00013581 | 0.00016053 |
| A0000009 | | 0.00000196 | 0.00000443 | 0.00000613 | 0.00000825 | 0.00001145 | 0.00001403 | 0.00001617 | 0.00001990 | 0.00002562 | 0.00003560 | 0.00004941 | 0.00006621 | 0.00008322 |

BANQUE DE DONNEES SPECTRALES AMELIOREE A'

FIGURE 4

EP 3 213 051 B1

EP 3 213 051 B1

| Absorbance Nom | %Poids | Germe 1 | Germe 2 | Germe 3 | 4764 | 4760 | 4756 | 4752 | 4748 | 4744 | 4740 | 4736 |
|---|---|---|---|---|---|---|---|---|---|---|---|---|
| A0000001 | | | | | 7.78E-06 | 1.29E-05 | 1.88E-05 | 2.42E-05 | 3.16E-05 | 4.42E-05 | 6.18E-05 | 8.01E-05 |
| A0000002 | | | | | 8.08E-06 | 1.37E-05 | 1.96E-05 | 2.5E-05 | 3.18E-05 | 4.46E-05 | 6.09E-05 | 7.91E-05 |
| A0000003 | | | | | 8.78E-06 | 1.52E-05 | 2.23E-05 | 2.97E-05 | 3.99E-05 | 5.53E-05 | 7.54E-05 | 9.68E-05 |
| A0000004 | | | | | 7.46E-06 | 1.26E-05 | 1.81E-05 | 2.47E-05 | 3.29E-05 | 4.71E-05 | 6.55E-05 | 8.47E-05 |
| A0000005 | | | | | 1.01E-05 | 1.59E-05 | 2.39E-05 | 3.28E-05 | 4.34E-05 | 5.93E-05 | 8.08E-05 | 0.000104 |
| A0000006 | | | | | 5.49E-06 | 9.2E-06 | 1.41E-05 | 1.88E-05 | 2.54E-05 | 3.62E-05 | 5.18E-05 | 6.97E-05 |
| A0000007 | | | | | 7.14E-06 | 1.21E-05 | 1.86E-05 | 2.41E-05 | 3.27E-05 | 4.63E-05 | 6.52E-05 | 8.49E-05 |
| A0000008 | | | | | 1.03E-05 | 1.64E-05 | 2.4E-05 | 3.26E-05 | 4.39E-05 | 6.13E-05 | 8.42E-05 | 0.000109 |
| A0000009 | | | | | 8.25E-06 | 1.15E-05 | 1.4E-05 | 1.62E-05 | 1.99E-05 | 2.56E-05 | 3.56E-05 | 4.94E-05 |
| I2G022 | 0.564<br>0.436 | A0000003 | A0000006 | | 7.35E-06 | 1.26E-05 | 1.88E-05 | 2.49E-05 | 3.36E-05 | 4.7E-05 | 6.51E-05 | 8.5E-05 |
| I2G011 | 0.654<br>0.346 | A0000009 | A0000001 | | 8.09E-06 | 1.2E-05 | 1.57E-05 | 1.9E-05 | 2.4E-05 | 3.21E-05 | 4.47E-05 | 6E-05 |
| I2G036 | 0.44<br>0.56 | A0000008 | A0000004 | | 8.69E-06 | 1.43E-05 | 2.07E-05 | 2.81E-05 | 3.77E-05 | 5.33E-05 | 7.38E-05 | 9.54E-05 |
| I3G038 | 0.747<br>0.258<br>-0.005 | A0000008 | A0000002 | A0000004 | 9.71E-06 | 1.57E-05 | 2.29E-05 | 3.06E-05 | 4.09E-05 | 5.7E-05 | 7.83E-05 | 0.000101 |
| I3G025 | 0.5825<br>0.3020<br>0.1155 | A0000008 | A0000005 | A0000003 | 1E-05 | 1.61E-05 | 2.38E-05 | 3.23E-05 | 4.33E-05 | 6E-05 | 8.22E-05 | 0.000106 |
| I3G019 | 0.094<br>-0.00047<br>0.00647 | A0000004 | A0000005 | A0000007 | 7.43E-06 | 1.25E-05 | 1.81E-05 | 2.46E-05 | 3.28E-05 | 4.7E-05 | 6.54E-05 | 8.46E-05 |

BANQUE DE DONNEES SPECTRALES ELARGIE E

FIGURE 5

| Absorbance Nom | Pole | Germe | %poids | 4764 | 4760 | 4756 | 4752 | 4748 | 4744 | 4740 | 4736 | 4732 |
|---|---|---|---|---|---|---|---|---|---|---|---|---|
| A0000001 | | | | 7.78E-06 | 1.29E-05 | 1.88E-05 | 2.42E-05 | 3.16E-05 | 4.42E-05 | 6.18E-05 | 8.01E-05 | 9.88E-05 |
| A0000002 | | | | 8.08E-06 | 1.37E-05 | 1.96E-05 | 2.5E-05 | 3.18E-05 | 4.46E-05 | 6.09E-05 | 7.91E-05 | 9.8E-05 |
| A0000003 | | | | 8.78E-06 | 1.52E-05 | 2.23E-05 | 2.97E-05 | 3.99E-05 | 5.53E-05 | 7.54E-05 | 9.68E-05 | 0.000118 |
| A0000004 | | | | 7.46E-06 | 1.26E-05 | 1.81E-05 | 2.47E-05 | 3.29E-05 | 4.71E-05 | 6.55E-05 | 8.47E-05 | 0.000104 |
| A0000005 | | | | 1.01E-05 | 1.59E-05 | 2.39E-05 | 3.28E-05 | 4.34E-05 | 5.93E-05 | 8.08E-05 | 0.000104 | 0.000128 |
| A0000006 | | | | 5.49E-06 | 9.2E-06 | 1.41E-05 | 1.88E-05 | 2.54E-05 | 3.62E-05 | 5.18E-05 | 6.97E-05 | 8.68E-05 |
| A0000007 | | | | 7.14E-06 | 1.21E-05 | 1.86E-05 | 2.41E-05 | 3.27E-05 | 4.63E-05 | 6.52E-05 | 8.49E-05 | 0.000104 |
| A0000008 | | | | 1.03E-05 | 1.64E-05 | 2.4E-05 | 3.26E-05 | 4.39E-05 | 6.13E-05 | 8.42E-05 | 0.000109 | 0.000136 |
| A0000009 | | | | 8.25E-06 | 1.15E-05 | 1.4E-05 | 1.62E-05 | 1.99E-05 | 2.56E-05 | 3.56E-05 | 4.94E-05 | 6.62E-05 |
| MEG001 | PAL054 | A0000009 | 0.15 | 4.45E-06 | 7.13E-06 | 9.35E-06 | 1.12E-05 | 1.44E-05 | 1.95E-05 | 2.84E-05 | 4.08E-05 | 5.62E-05 |
| MEG002 | PAL014 | A0000005 | -0.05 | 1.15E-05 | 1.78E-05 | 2.63E-05 | 3.57E-05 | 4.69E-05 | 6.36E-05 | 8.61E-05 | 0.00011 | 0.000135 |
| MEG003 | PAL035 | A0000008 | 0.08 | 8.11E-06 | 1.37E-05 | 2.06E-05 | 2.84E-05 | 3.88E-05 | 5.48E-05 | 7.6E-05 | 9.89E-05 | 0.000124 |
| MEG004 | PRF006 | A0000002 | 0.021655 | 8.65E-06 | 1.43E-05 | 2.03E-05 | 2.57E-05 | 3.26E-05 | 4.53E-05 | 6.16E-05 | 7.98E-05 | 9.88E-05 |
| MEG005 | PRF004 | A0000007 | -0.07268 | 4.86E-06 | 9.48E-06 | 1.56E-05 | 2.07E-05 | 2.94E-05 | 4.35E-05 | 6.29E-05 | 8.26E-05 | 0.000102 |
| MEG006 | PRF074 | A0000003 | 0.028752 | 9.54E-06 | 1.61E-05 | 2.33E-05 | 3.07E-05 | 4.07E-05 | 5.59E-05 | 7.58E-05 | 9.72E-05 | 0.000119 |

BANQUE DE DONNEES SPECTRALES ELARGIE EE

FIGURE 6

EP 3 213 051 B1

| Absorbance Nom | Germe1 | Germe2 | Germe3 | pole | %poids | 4764 | 4760 | 4756 | 4752 | 4748 | 4744 | 4740 |
|---|---|---|---|---|---|---|---|---|---|---|---|---|
| A0000001 | | | | | | 7.78E-06 | 1.29E-05 | 1.88E-05 | 2.42E-05 | 3.16E-05 | 4.42E-05 | 6.18E-05 |
| A0000002 | | | | | | 8.08E-06 | 1.37E-05 | 1.96E-05 | 2.5E-05 | 3.18E-05 | 4.46E-05 | 6.09E-05 |
| A0000003 | | | | | | 8.78E-06 | 1.52E-05 | 2.23E-05 | 2.97E-05 | 3.99E-05 | 5.53E-05 | 7.54E-05 |
| A0000004 | | | | | | 7.46E-06 | 1.26E-05 | 1.81E-05 | 2.47E-05 | 3.29E-05 | 4.71E-05 | 6.55E-05 |
| A0000005 | | | | | | 1.01E-05 | 1.59E-05 | 2.39E-05 | 3.28E-05 | 4.34E-05 | 5.93E-05 | 8.08E-05 |
| A0000006 | | | | | | 5.49E-06 | 9.2E-06 | 1.41E-05 | 1.88E-05 | 2.54E-05 | 3.62E-05 | 5.18E-05 |
| A0000007 | | | | | | 7.14E-06 | 1.21E-05 | 1.86E-05 | 2.41E-05 | 3.27E-05 | 4.63E-05 | 6.52E-05 |
| A0000008 | | | | | | 1.03E-05 | 1.64E-05 | 2.4E-05 | 3.26E-05 | 4.39E-05 | 6.13E-05 | 8.42E-05 |
| A0000009 | | | | | | 8.25E-06 | 1.15E-05 | 1.4E-05 | 1.62E-05 | 1.99E-05 | 2.56E-05 | 3.56E-05 |
| MEP001 | A0000002 | A0000006 | | 0 PAL031 | 0.56 / 0.34 / 0.1 | 4.86E-06 | 8.85E-06 | 1.35E-05 | 1.8E-05 | 2.41E-05 | 3.54E-05 | 5.04E-05 |
| MEP002 | A0000005 | A0000003 | | 0 PRF028 | 0.304 / 0.646 / 0.05 | 1.05E-05 | 1.68E-05 | 2.45E-05 | 3.24E-05 | 4.26E-05 | 5.78E-05 | 7.8E-05 |
| MEP003 | A0000005 | A0000008 | | 0 PRF063 | 1.1726 / -0.0926 / -0.08 | 8.68E-06 | 1.44E-05 | 2.21E-05 | 3.1E-05 | 4.19E-05 | 5.78E-05 | 7.97E-05 |
| MEP004 | A0000006 | A0000009 | A0000002 | PAL037 | 0.306 / -0.0530 / 0.647 / 0.1 | 4.89E-06 | 9.38E-06 | 1.46E-05 | 1.93E-05 | 2.55E-05 | 3.67E-05 | 5.17E-05 |
| MEP005 | A0000008 | A0000005 | A0000003 | PAL006 | 0.6062 / 0.314 / 0.1198 / -0.04 | 1.11E-05 | 1.76E-05 | 2.56E-05 | 3.44E-05 | 4.59E-05 | 6.33E-05 | 8.63E-05 |
| MEP006 | A0000002 | A0000006 | A0000005 | PRF025 | 0.273 / 0.4170 / 0.22 / 0.09 | 9.96E-06 | 1.52E-05 | 2.16E-05 | 2.8E-05 | 3.54E-05 | 4.75E-05 | 6.44E-05 |

BANQUE DE DONNEES SPECTRALES ELARGIE EEI

FIGURE 7

*FIG. 8*

EP 3 213 051 B1

| Name | Kcy | Ksatu | Karo | Kiso | Kene | xxxx |
|---|---|---|---|---|---|---|
| CRK0071 | 124.2982 | 30.2213 | 6.1022 | 20.4044 | 22.6714 | |
| CRK0075 | 123.6416 | 30.3821 | 6.0959 | 20.0519 | 22.6293 | |
| CRK0098 | 123.3631 | 29.719 | 6.1168 | 21.0968 | 22.9267 | |
| CRK0102 | 122.6272 | 29.1777 | 6.1166 | 20.8441 | 23.3606 | |
| CRK0116 | 120.3105 | 29.5099 | 6.1134 | 21.0583 | 23.0629 | |
| HVY0068 | 144.4259 | 52.0212 | 5.9475 | 22.2027 | 13.5996 | |
| HVY0088 | 141.8204 | 47.8997 | 6.0091 | 23.0564 | 14.2034 | |
| HVY0093 | 143.0184 | 49.2953 | 5.9834 | 22.8627 | 14.0157 | |
| HVY0100 | 142.1157 | 48.486 | 5.9932 | 22.8486 | 14.183 | |
| HVY0106 | 143.3684 | 50.5179 | 5.9642 | 22.8179 | 13.8181 | |
| KER0072 | 114.2108 | 61.7041 | 5.8061 | 22.7724 | 13.9045 | |
| KER0076 | 112.7754 | 59.6154 | 5.8289 | 23.7607 | 14.191 | |
| KER0079 | 113.9782 | 56.389 | 5.8904 | 22.7878 | 14.299 | |
| KER0080 | 113.2538 | 57.5513 | 5.8681 | 23.0583 | 14.2554 | |
| KER0082 | 114.2399 | 62.3519 | 5.8108 | 23.6506 | 13.8976 | |
| LGT0067 | 130.8834 | 53.1217 | 5.9227 | 22.969 | 13.9002 | |
| LGT0087 | 129.4485 | 49.2411 | 5.9723 | 23.7952 | 14.4472 | |
| LGT0096 | 129.5462 | 48.6752 | 5.9809 | 23.382 | 14.5025 | |
| LGT0099 | 129.1717 | 48.8969 | 5.9775 | 23.6349 | 14.5186 | |
| LGT0105 | 129.4355 | 50.1752 | 5.9592 | 23.4217 | 14.267 | |

Tableau d'Agrégats

FIGURE 9

EP 3 213 051 B1

| Absorbance Nom | %Poids | Germe 1 | Germe 2 | Germe 3 | 4764 | 4760 | 4756 | 4752 | 4748 | 4744 | 4740 | 4736 | 4732 | MON | RON |
|---|---|---|---|---|---|---|---|---|---|---|---|---|---|---|---|
| A0000001 | | | | | 7.78E-06 | 1.29E-05 | 1.88E-05 | 2.42E-05 | 3.16E-05 | 4.42E-05 | 6.18E-05 | 8.01E-05 | 9.88E-05 | 85.1 | 94.2 |
| A0000002 | | | | | 8.08E-06 | 1.37E-05 | 1.96E-05 | 2.5E-05 | 3.18E-05 | 4.46E-05 | 6.09E-05 | 7.91E-05 | 9.8E-05 | 85.3 | 94.7 |
| A0000003 | | | | | 8.78E-06 | 1.52E-05 | 2.23E-05 | 2.97E-05 | 3.99E-05 | 5.53E-05 | 7.54E-05 | 9.68E-05 | 0.000118 | 85 | 94 |
| A0000004 | | | | | 7.46E-06 | 1.26E-05 | 1.81E-05 | 2.47E-05 | 3.29E-05 | 4.71E-05 | 6.55E-05 | 8.47E-05 | 0.000104 | 85 | 93.5 |
| A0000005 | | | | | 1.01E-05 | 1.59E-05 | 2.39E-05 | 3.28E-05 | 4.34E-05 | 5.93E-05 | 8.08E-05 | 0.000104 | 0.000128 | 85.3 | 95.1 |
| A0000006 | | | | | 5.49E-06 | 9.2E-06 | 1.41E-05 | 1.88E-05 | 2.54E-05 | 3.62E-05 | 5.18E-05 | 6.97E-05 | 8.68E-05 | 84.4 | 92.8 |
| A0000007 | | | | | 7.14E-06 | 1.21E-05 | 1.86E-05 | 2.41E-05 | 3.27E-05 | 4.63E-05 | 6.52E-05 | 8.49E-05 | 0.000104 | 85 | 93.5 |
| A0000008 | | | | | 1.03E-05 | 1.64E-05 | 2.4E-05 | 3.26E-05 | 4.39E-05 | 6.13E-05 | 8.42E-05 | 0.000109 | 0.000136 | 85.5 | 95.1 |
| A0000009 | | | | | 8.25E-06 | 1.15E-05 | 1.4E-05 | 1.62E-05 | 1.99E-05 | 2.56E-05 | 3.56E-05 | 4.94E-05 | 6.62E-05 | 85.4 | 95 |
| I2G022 | 0.564 0.436 | A0000003 | A0000006 | | 7.35E-06 | 1.26E-05 | 1.88E-05 | 2.49E-05 | 3.36E-05 | 4.7E-05 | 6.51E-05 | 8.5E-05 | 0.000105 | 84.73846 | 93.47693 |
| I2G011 | 0.654 0.346 | A0000009 | A0000001 | | 8.09E-06 | 1.2E-05 | 1.57E-05 | 1.9E-05 | 2.4E-05 | 3.21E-05 | 4.47E-05 | 6E-05 | 7.75E-05 | 85.29608 | 94.72287 |
| I2G036 | 0.44 0.56 | A0000008 | A0000004 | | 8.69E-06 | 1.43E-05 | 2.07E-05 | 2.81E-05 | 3.77E-05 | 5.33E-05 | 7.38E-05 | 9.54E-05 | 0.000118 | 85.21994 | 94.20381 |
| I3G038 | 0.747 0.258 -0.005 | A0000008 | A0000002 | A0000004 | 9.71E-06 | 1.57E-05 | 2.29E-05 | 3.06E-05 | 4.09E-05 | 5.7E-05 | 7.83E-05 | 0.000101 | 0.000126 | 85.45096 | 95.00502 |
| I3G025 | 0.5825 0.3020 0.1155 | A0000008 | A0000005 | A0000003 | 1E-05 | 1.61E-05 | 2.38E-05 | 3.23E-05 | 4.33E-05 | 6E-05 | 8.22E-05 | 0.000106 | 0.000131 | 85.38181 | 94.97284 |
| I3G019 | 0.094 -0.00047 0.00647 | A0000004 | A0000005 | A0000007 | 7.43E-06 | 1.25E-05 | 1.81E-05 | 2.46E-05 | 3.28E-05 | 4.7E-05 | 6.54E-05 | 8.46E-05 | 0.000104 | 84.99859 | 93.49245 |

BANQUE DE DONNEES SPECTRALES ELARGIE E – avec caractérisation

FIGURE 10

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2010116991 A
- US 6087662 A
- WO 2011073855 A

- US 6070128 A
- US 20040033617 A1
- EP 0742900 A